(19) Europäisches Patentamt
European Patent Office
Office européen des brevets

(11) **EP 3 831 290 A1**

(12) **EUROPEAN PATENT APPLICATION**
published in accordance with Art. 153(4) EPC

(43) Date of publication:
**09.06.2021 Bulletin 2021/23**

(51) Int Cl.:
*A61B 5/055* (2006.01)   *A61B 6/03* (2006.01)
*A61B 10/00* (2006.01)   *G06Q 50/22* (2018.01)

(21) Application number: **19845536.2**

(22) Date of filing: **31.07.2019**

(86) International application number:
**PCT/JP2019/030056**

(87) International publication number:
**WO 2020/027213 (06.02.2020 Gazette 2020/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(30) Priority: **31.07.2018 JP 2018143199**

(71) Applicant: **Splink, Inc.**
**Tokyo100-0013 (JP)**

(72) Inventors:
• **AOYAMA, Yuki**
  **Tokyo 100-0013 (JP)**
• **KASAI, Wataru**
  **Tokyo 100-0013 (JP)**

(74) Representative: **Körber, Martin Hans**
  **Mitscherlich PartmbB**
  **Patent- und Rechtsanwälte**
  **Sonnenstrasse 33**
  **80331 München (DE)**

(54) **DEMENTIA RISK PRESENTATION SYSTEM AND METHOD**

(57)     There is provided a dementia risk presentation system (10) that presents a dementia risk of a user. The presentation system includes: a first database (17) in which sample brain health data (17a), including at least one type of data for sample brain state data (17d) that are data relating to states of brains of sample subjects and sample cognition data (17e) that is data relating to cognitive ability which is a function of the brains, are associated with individual characteristics (17b) including at least one of an age, gender and physical information of each sample subject; a first recognition unit (11) that recognizes user brain health data (18a) including at least one type of data for user brain state data (41) and user cognition data (61); a second recognition unit (12) that recognizes user individual characteristics (43); and a risk presentation unit (30) that presents a chronological transition line (52) indicating transitions in the user brain health data of the user from the past to the present and a risk line (54) relating to dementia.

Fig. 1

**Description**

Technical Field

The present invention relates to a system and method that present the dementia risk of a user.

Background Art

Japanese Laid-open Patent Publication No. 2016-22310 discloses the provision of a dementia risk determination system that is capable of determining the potential risk of dementia at an extremely early stage and providing an opportunity for prevention to delay the onset of dementia. This system is equipped with a biometric data detection sensor that acquires biometric data of the subject during sleep, a sleep data generation apparatus that generates sleep data, including the depth of sleep of the subject over time and changes in body movements, from the biometric data of the subject acquired by the biometric data detection sensor, and a dementia risk determination apparatus that includes a storage unit that stores, for predetermined symptoms relating to dementia, sleep data specific to each symptom obtained from actual onset patients. The dementia risk determination apparatus compares the sleep data of the subject generated by the sleep data generation apparatus with the sleep data for the respective symptoms stored in the storage unit and determines three dementia risks from the sleep data of the subject.

Summary of Invention

It is known that at the initial stage of dementia onset (which includes not only actual onset but also a state where onset is suspected), it is possible to suppress progression by giving appropriate treatment or making appropriate changes to lifestyle habits such as exercise and alcohol consumption. This makes it desirable to identify the possibility of dementia onset or the degree of progression after onset or occurrence at the earliest possible stage. In response to this, various systems for identifying the risk of onset and further progression of dementia (hereinafter, simply referred to as "dementia risk" or "risk of dementia") have been proposed.

In the system for determining the dementia risk from sleep data described above, the functioning of the user's brain is estimated according to the state during sleep, and the dementia risk is recognized based on this estimation result. This means that the accuracy of the recognized dementia risk is affected by the accuracy of the estimation, which results in a problem in that it is difficult to appropriately recognize dementia risk. There is also a problem that it is difficult for a person who does not have sufficient knowledge to properly understand what the dementia risk assessment is, when presented with information simply indicating the presence or absence of a dementia risk. A system and method that can accurately recognize dementia risk and can present this information in an easy-to-understand format is therefore needed.

One aspect of the present invention is a system including: a first database that includes sample brain health data of sample subjects (test subjects) associated with individual characteristics of the sample subjects, wherein the individual characteristics include at least one of age, gender, and physical information of the sample subjects respectively, the sample brain health data includes at least one type of data for sample brain state data that is data pertaining to states of brains of sample subjects and sample cognition data that is data pertaining to cognitive ability as functions of the brains of the sample subject. The system further includes a first recognition unit that recognizes user brain health data including at least one type of data for user brain state data, which is data relating to a state of a brain of a user, and user cognition data, which is data relating to cognitive ability as a function of the brain of the user; a second recognition unit that recognizes a user individual characteristic indicating individual characteristics of the user; and a risk presentation unit that presents a chronological transition line and a risk line relating to dementia. The chronological transition line (aging line) indicates transitions in the user brain health data of the user from the past to the present based on past and present user brain health data of the user, and the risk line is derived by referring to transitions according to age in the sample brain health data associated with the individual characteristics corresponding to the user individual characteristic.

In this system, the dementia risk is recognized by comparing user brain health data (data on the health state of the brain), which includes at least one type of data for user brain state data relating to the brain state of the user and user cognition data relating to cognitive ability that is a function of the brain of the user whose risk is to be determined, and data which relates to the health state of the brain (sample brain health data) of the sample subjects that includes at least one type of data similarly that of the user brain health data. The data pertaining to brain state are the data directly indicating the condition (physical condition) of the brain in the form of quantitative parameters relating to the state (physical state) of the brain that affects cognitive ability and that are different from the indirect data showing the brain function that are inferred from the user's brain waves, behavior, movements, and the like. Cognitive ability tests, which acquire the cognition data (cognitive ability data), include contents that are enhanced as criteria for determining dementia risk, and can accurately determine the conditions of brain functions. Examples of cognitive tests are described in the Japanese Patent Application No. 2018-33652 filed by the present Applicant. Accordingly, the dementia risk can be accurately recognized by comparing the user brain health data including one or both types of information with the sample brain health data.

In addition, by presenting the dementia risk in formats of a chronological transition line (transition on time line, aging line) and risk line, both lines showing conditions according to age (in keeping with age) of the user for comparing, it is possible for the user and others to grasp not only the scale of the dementia risk of the user but simultaneously also the trend in the dementia risk of the user (such as whether the risk is worsening, improving, or deterioration is being suppressed). This means that it is possible for the user or a person who manages the health of the user (hereinafter, collectively referred to as "the user and others") to intuitively grasp how high or low the dementia risk is, from the information presented by this system, even when the user and others do not have sufficient knowledge for dementia.

The risk presentation unit may include a first score presentation unit that presents a score indicating dementia risk of the user or a symbol corresponding to the score based on a result of a comparison between the user brain health data and the sample brain health data associated with individual characteristics corresponding to the user individual characteristic. By presenting the dementia risk as a score (first type score) or a symbol corresponding to (in keeping with) a score (hereinafter referred to as a "score or the like"), it is possible to the user and others to intuitively figure out how high or low the dementia risk of the user is.

The system may further include a prediction unit that predicts a chronological prediction line (prediction on time line, aging prediction line) indicating predicted transitions in the user brain health data for the user predicted from the present to the future based on the chronological transition line, and the risk presentation unit may include a prediction presentation unit that presents the chronological prediction line in addition to the chronological prediction line and the risk line. By showing the risk line and the chronological prediction line, which is a prediction of transitions in the dementia risk from the present to the future so as to allow comparison, it is possible for the user and others to grasp the trend in the dementia risk of the user more accurately.

The risk presentation unit may include a second score presentation unit that presents, together with the chronological prediction line or in place of the chronological prediction line, a score (second type score) indicating a future dementia risk of the user or a symbol corresponding to the score, based on a result of a comparison between the user brain health data predicted at a first age on the chronological prediction line and the sample brain health data associated with individual characteristics corresponding to an individual characteristic obtained by including the first age along with the user individual characteristic. The risk presentation unit may predict or forecast user brain health data of the user for the first age in the future in addition to the chronological prediction line, and may present a score indicating the future dementia risk or a symbol corresponding to the score based on the result of a comparison between the predicted user brain health data and sample brain health data associated with the individual characteristics corresponding to the individual characteristic obtained by including the first age along with the user individual characteristic.

In the first database, the sample brain health data of the sample subjects may be associated with the individual characteristics and with lifestyle characteristics of the sample subjects, wherein lifestyle characteristics include at least one of lifestyle habits and living environments of the sample subjects. The system may further include a third recognition unit that recognizes user lifestyle characteristic indicating lifestyle characteristics of the user, and the prediction unit may predict the chronological prediction line based on the chronological transition line and the sample brain health data associated with individual characteristics corresponding to the user individual characteristic, the lifestyle characteristics corresponding to the user lifestyle characteristic, and age of the user. By referring to the user lifestyle characteristic when recognizing the chronological prediction line, it is possible to recognize a highly reliable chronological prediction line (and in turn, the trend in the dementia risk). By doing so, it is possible for the user and others to grasp the trend in the dementia risk of the user even more accurately.

The risk presentation unit may include a third score presentation unit that presents, together with or instead of the chronological prediction line, a score (third type score) indicating the future dementia risk of the user or a symbol corresponding to the score, based on a result of a comparison between the user brain health data predicted at the first age on the chronological prediction line and the sample brain health data associated with individual characteristics corresponding to the individual characteristic obtained by including the first age along with the user individual characteristic and the lifestyle characteristics corresponding to the user lifestyle characteristic. The risk presentation unit may, in addition to the chronological prediction line, predict user brain health data corresponding to the user lifestyle characteristic that has been specified by the user or have been arbitrarily selected, and may present the score indicating the future dementia risk of the user or a symbol in keeping with the score based on the result of a comparison between the predicted user brain health data and sample brain health data associated with lifestyle characteristics corresponding to the user lifestyle characteristic.

The prediction unit may include a comparison/prediction unit (comparison and prediction unit, comparison prediction unit) that recognizes a first chronological prediction line based on a first user lifestyle characteristic and a second chronological prediction line based on a second user lifestyle characteristic for the identical user, and the risk presentation unit may include a comparison/presentation unit (comparison and presentation unit, comparison presentation unit) that presents the first chronological prediction line and the second chronological prediction line side by side or consecutively to allow comparison. The user and others can intuitively grasp variations in the chronological prediction line (and in turn in the dementia risk) in keeping with variations in the lifestyle characteristics. By doing so, the user and others can grasp lifestyle characteristics that are to be improved in more specific terms, and an incentive is provided to improve the lifestyle characteristics.

The risk presentation unit may include a fourth score presentation unit that presents a first score indicating a future dementia risk of the user corresponding to a first chronological prediction line and a second score indicating a future dementia risk of the user corresponding to a second chronological prediction line. The respective scores are calculated based on the results of comparisons between the user brain health data of a first age on the respective chronological prediction lines and sample brain health data associated with the individual characteristics corresponding to the individual characteristics obtained by including the first age along with the user individual characteristic and the lifestyle characteristics corresponding to the respective user lifestyle characteristics. The risk presentation unit may predict the future dementia risk of the user based on the respective user lifestyle characteristics in addition to the chronological prediction lines, and present the first score and the second score indicating the respective risks.

The sample brain state data and the user brain state data may include at least one type of data for indicating a volume of an entire brain, a volume of at least one predetermined region of the brain, brain images, cerebral blood flow, and electroencephalograms, and may include information capable of directly or indirectly indicating the state of the brain, for example, information on blood biomarkers.

Another aspect of the present invention is a method of presenting dementia risk. This method uses a system capable of referring to a first database in which sample brain health data of the sample subjects, including at least one type of data for sample brain state data that are data pertaining to states of brains of sample subjects and sample cognition data that are data relating to cognitive ability which is a function of the brain, are associated with individual characteristics including at least one of an age, gender, and physical information of each sample subject, and includes the following steps.

1. Recognizing user brain health data including at least one type of data for user brain state data, which is data relating to a state of a brain of a user, and user cognition data, which is data relating to cognitive ability which is a function of the brain of the user.
2. Recognizing user individual characteristics indicating individual characteristics of the user.
3. Presenting a chronological transition line and a risk line relating to dementia, wherein the chronological transition line indicates transitions in the user brain health data of the user from the past to the present based on past and present user brain health data of the user, and the risk line is derived by referring to transitions according to age in the sample brain health data associated with the individual characteristics corresponding to the user individual characteristic.

The method may further include presenting a score indicating dementia risk of the user or a symbol corresponding to the score based on a result of a comparison between the user brain health data and the sample brain health data associated with individual characteristics corresponding to the user individual characteristic.

The method may further include predicting a chronological prediction line indicating predicted transitions of the user brain health data for the user predicted from the present to the future based on the chronological line, and presenting the risk may include presenting the chronological prediction line in addition to the chronological transition line and the risk line. The method may also include presenting, together with the chronological prediction line or in place of the chronological prediction line, a score indicating a future dementia risk of the user or a symbol corresponding to the score, based on a result of a comparison between the user brain health data at a first age on the chronological prediction line and the sample brain health data associated with individual characteristics corresponding to an individual characteristic obtained by including the first age along with the user individual characteristic.

In the first database, the sample brain health data of the sample subjects may be associated with the individual characteristics and with lifestyle characteristics of the sample subjects. The lifestyle characteristics may include at least one of lifestyle habits and living environments of the sample subjects. The method may further include recognizing a user lifestyle characteristic, indicating lifestyle characteristics of the user, and the predicting may include predicting the chronological prediction line based on the chronological transition line and the sample brain health data associated with the individual characteristics corresponding to the user individual characteristic, the lifestyle characteristics corresponding to the user lifestyle characteristic, and age of the user.

The method may further include presenting, together with or instead of the chronological prediction line, a score indicating the future dementia risk of the user or a symbol corresponding to the score, based on a result of a comparison between the user brain health data at a first age on the chronological prediction line and the sample brain health data associated with individual characteristics corresponding to an individual characteristic obtained by including the first age along with the user individual characteristic and the lifestyle characteristics corresponding to the user lifestyle characteristic. This method may predict, separately to the chronological prediction line, user brain health data of a first age that is specified by the user or is arbitrarily selected, and present a score indicating the future dementia risk of the user or a symbol in keeping with the score based on a result of a comparison between the predicted user brain health data and sample brain health data associated with individual characteristics corresponding to an individual characteristic obtained by including the first age along with the user individual characteristic and lifestyle characteristics corresponding to the user lifestyle characteristic.

The predicting may include predicting a first chronological prediction line based on a first user lifestyle characteristic and a second chronological prediction line based on the second user lifestyle characteristic for the identical user, and the presenting of the risk may include presenting the first chronological prediction line and the second chronological prediction line side by side or consecutively to allow comparison. The method may further include presenting a score for a first age on the first chronological prediction line and a score for the first age on the second chronological prediction line. The method may present a plurality of scores relating to dementia risks that have been predicted based on user lifestyle characteristics that are arbitrary or have been specified by the user.

Another aspect of the present invention is a program or program product including instructions that cause a computer to operate as the system described above. This program or program product may be provided by being recorded on any type of recording or storage medium.

Brief Description of Drawings

[Fig. 1]

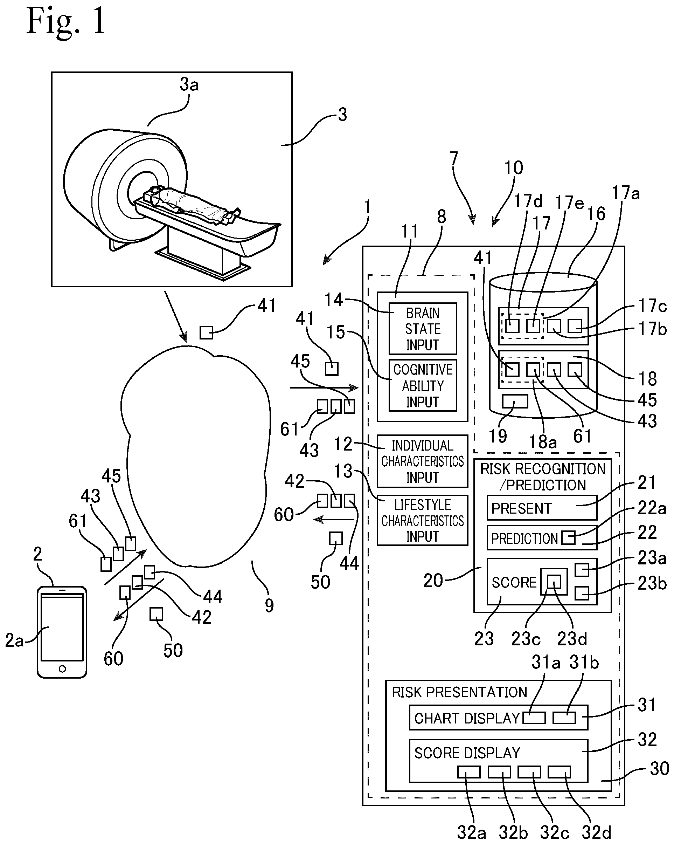

Fig. 1 depicts the overall configuration of a dementia risk presentation system.
[Fig. 2]

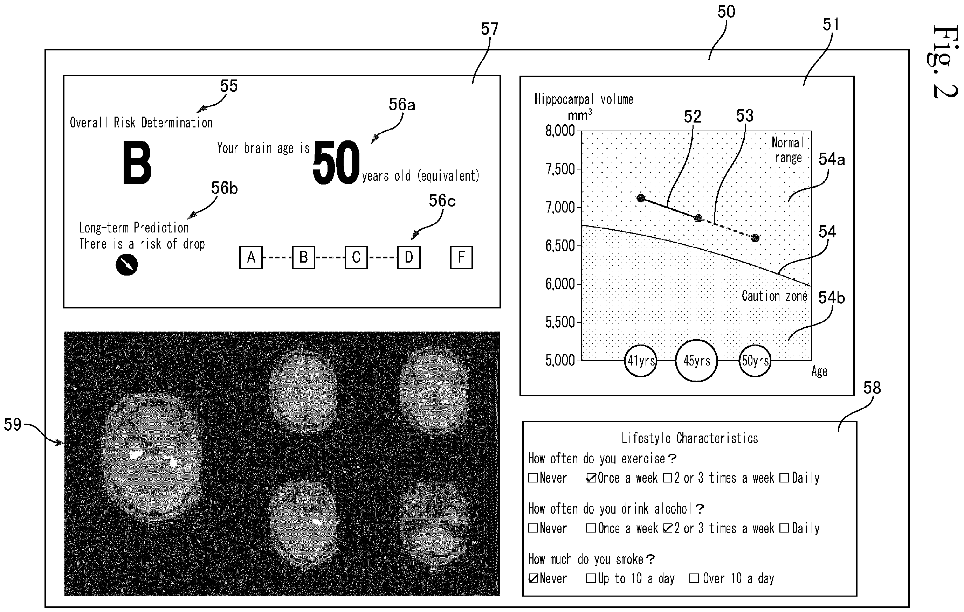

Fig. 2 depicts one example of a report presented by the presentation system.
[Fig. 3]

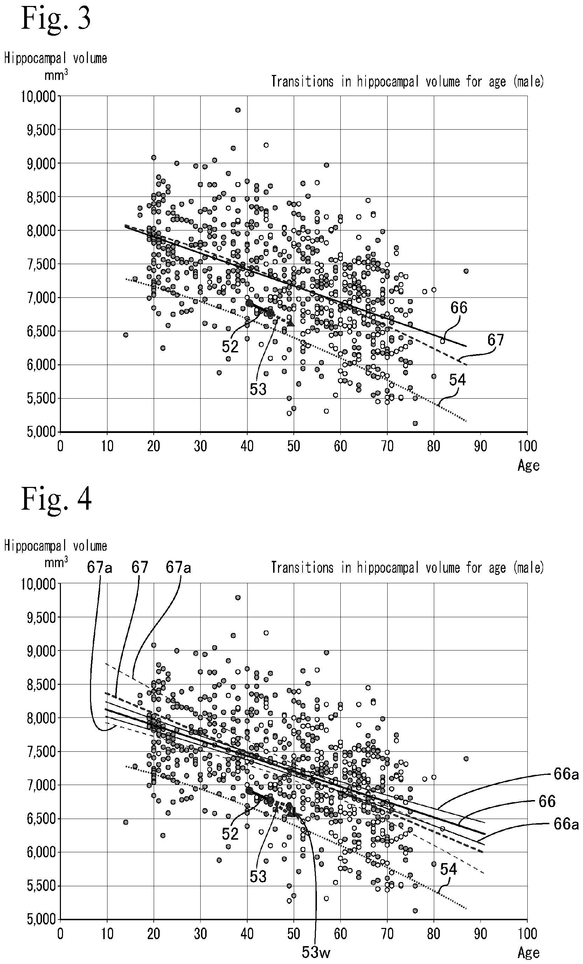

Fig. 3 is a graph in which the brain state data referred to when presenting dementia risk as chronological transitions has been plotted, with the vertical axis indicating hippocampal volume and the horizontal axis indicating age.
[Fig. 4]
Fig. 4 is a graph in which brain state data has been plotted in the same way as Fig. 3 and also depicts the range of a 95% CI.
[Fig. 5]

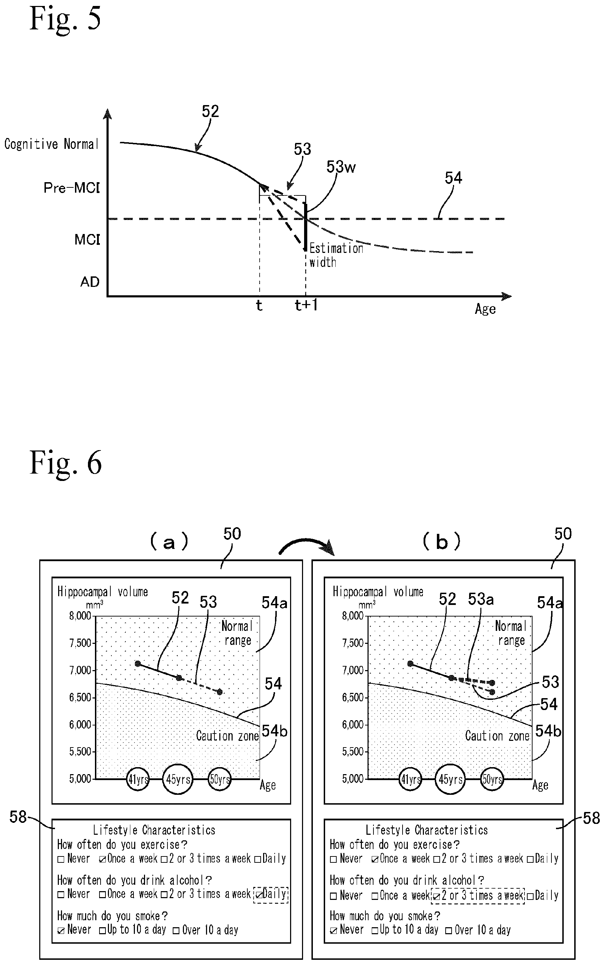

Fig. 5 is an example output including a chronological prediction line provided with an estimation width.
[Fig. 6]
Fig. 6 is an example indicating variation in the content of the report when the lifestyle characteristics are changed, where Fig. 6(a) depicts a case of daily alcohol consumption, and Fig. 6(b) depicts a case of alcohol consumption two or three days a week.
[Fig. 7]

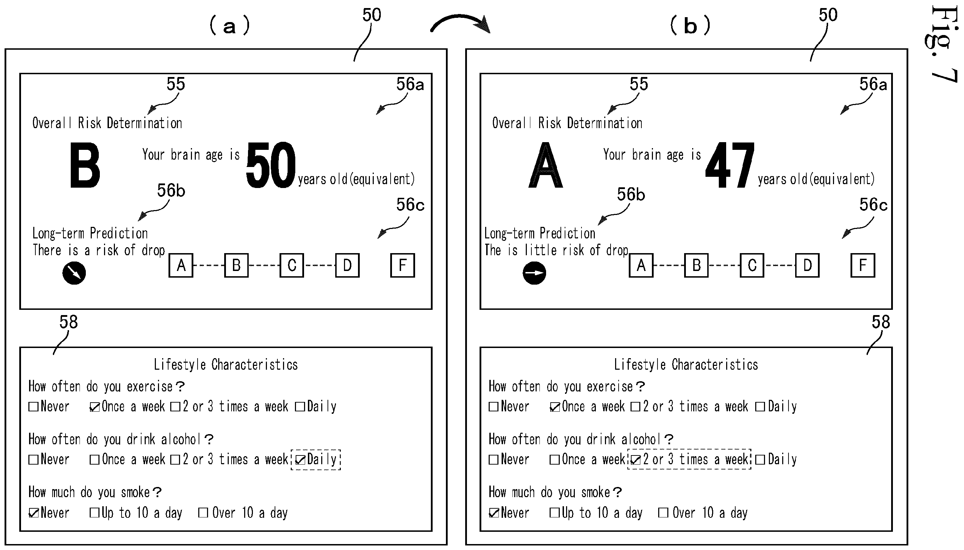

Fig. 7 is another example indicating variation in the display content of the report when the lifestyle characteristics are changed, where Fig. 7(a) depicts a case of drinking alcohol every day, and Fig. 7(b) depicts a case of drinking alcohol two or three days a week.
[Fig. 8]

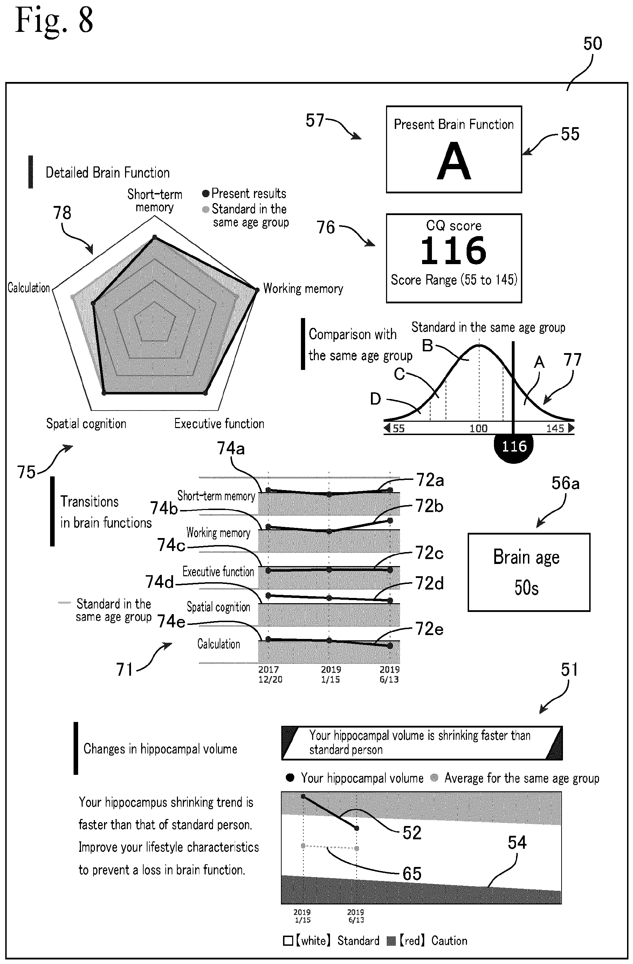

Fig. 8 is a diagram depicting another example of a report provided from the presentation system.
[Fig. 9]

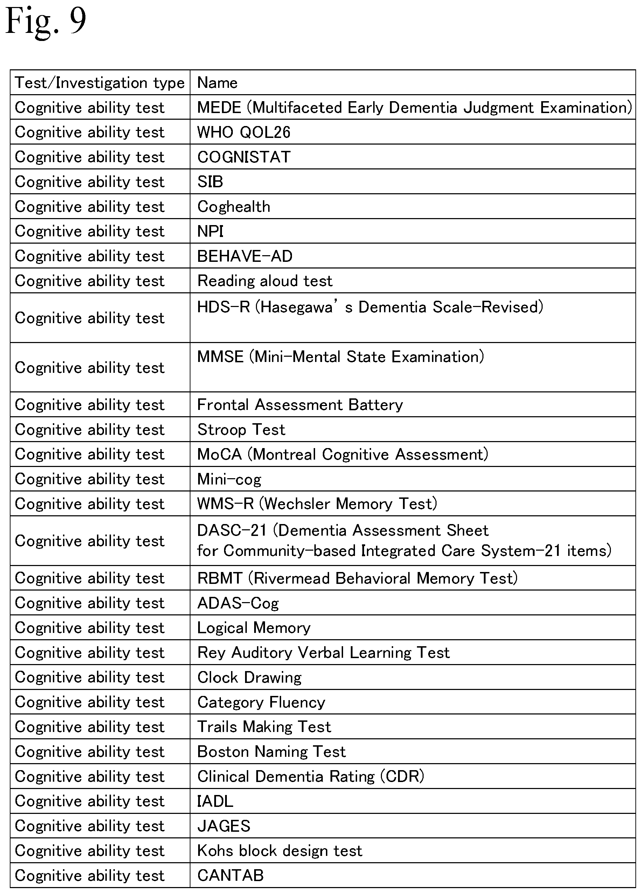

Fig. 9 is a diagram depicting examples of tests and investigations that measure the health state of the brain.
[Fig. 10]

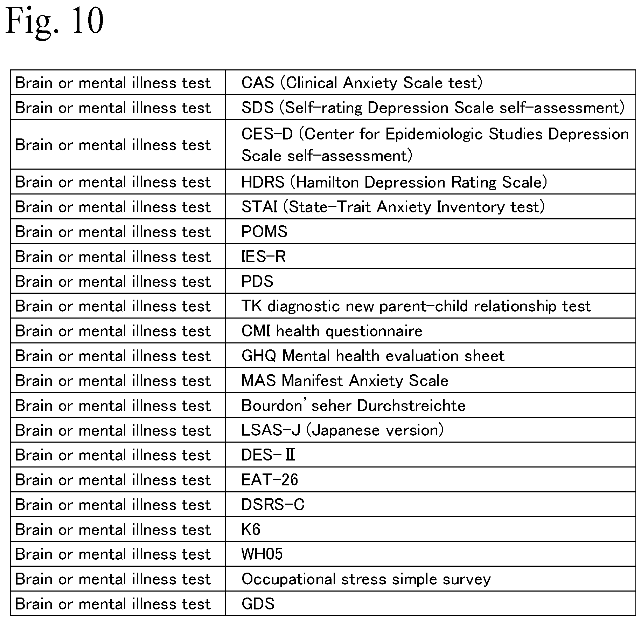

Fig. 10 is a diagram depicting examples of tests and investigations that measure the health state of the brain continuing from Fig. 9.
[Fig. 11]

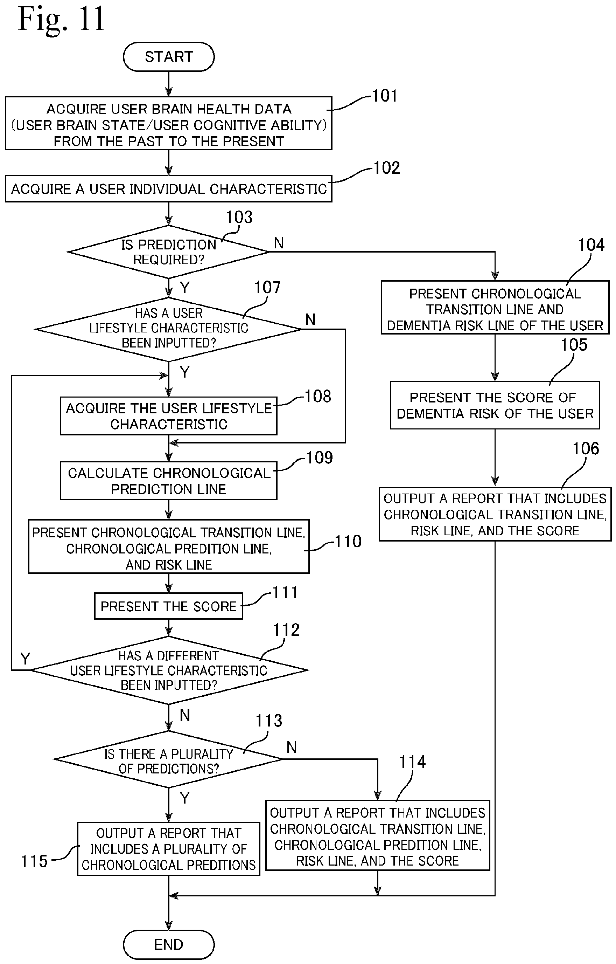

Fig. 11 is a flowchart depicting an overview of the operation of the presentation system.

Description of Embodiments

Fig. 1 depicts an example of a system that includes an apparatus (or system) that presents dementia risk. This system 1 includes a hospital 3 with an MRI (Magnetic Resonance Imaging) device (MRI) 3a, an information terminal (personal computer or tablet terminal) 2 that inputs various information about a user of the information terminal and outputs (displays) a report 50 relating to dementia risk, and an information providing apparatus (dementia risk presentation apparatus, dementia risk presentation system, dementia risk report providing system, or simply presentation system) 10 that processes information received from the MRI 3a and the tablet 2 via the Internet 9 and transmits the report 50, which includes dementia risk and other information. In the present embodiment, the presentation system 10 includes computer resources, such as a memory and a CPU, and is implemented on a server 7 capable of inputting and outputting information via the Internet (or cloud) 9. However, it is also possible for the presentation system 10 to be integrated with or implemented in the information terminal 2 of the user, to be connected using wires or wirelessly to the user terminal 2, or to be installed inside the hospital 3. Some or all of the functions of the presentation system 10 may be distributed and implemented across a plurality of information processing apparatuses including the user terminal 2.

The MRI 3a acquires data related to the state of the user's brain (user brain state data) and transmits the user brain state data 41 to the presentation system 10. The device for acquiring the user brain state data 41 is not limited to the MRI 3a, and may be any device configured to recognize user brain state data of the user that is the target (target user) for determining the dementia risk. As examples, a CT (Computed Tomography) inspection apparatus, an ultrasound inspection apparatus, an apparatus such as FDG or SPECT that measures brain function (cerebral blood flow), or an apparatus for measuring brain waves, blood biomarkers, or the like may be used, and information that can be measured by such apparatuses may be included in the data relating to the brain state. Also, an amount of a predetermined protein in the brain may be used as the data relating to the brain state in place of the shaped and/or volume of the brain, such as a PET (Positron Emission Tomography) image acquisition apparatus or the like may be used. The presentation system 10 may use brain state data 41 obtained from the MRI 3a, or may use brain state data 41 that were obtained in advance and stored in a user library 18.

Although an example where a value of hippocampal volume is used as the brain state data 41 is described below, the brain state data 41 is not limited to this. Here, the expression "brain state" refers to the state of the entire brain, or the state of regions of the brain that affect cognitive ability (as examples, the hippocampus, the supramarginal gyrus, the angular gyrus, the superior frontal gyrus, the middle frontal gyrus, the inferior frontal gyrus, and the anterior cingulate cortex). As examples, the expression "state" here may refer to or include the volume and shape of the entire brain or predetermined region(s) of the brain and/or the amount of a predetermined protein (as one example, amyloid $\beta$) in the brain. The brain state data (data relating to the brain state or data relating to the state of the brain) 41 includes quantitative parameters based on the state of the brain. As examples, the brain state data 41 may include the state of the brain itself (that is, the shape of the entire brain or predetermined regions) or numerical values representing the volume or size of the brain. The brain state data 41 may relate to a single brain state or may relate to a plurality of brain states. As specific examples, the brain state data 41 may include a value for the hippocampus or may include a value obtained by a predetermined formula based on the value for the hippocampus and the amount of amyloid $\beta$ in the brain.

The tablet 2 that inputs information about the user into the presentation system 10 and receives the report 50 including information on the dementia risk of the user from the presentation system 10 is equipped with a touch panel 2a (input/output unit). The touch panel 2a is used to input (and change) individual characteristics 43 of the user in response to an individual characteristics enquiry 42 from the presentation system 10 and to input (and change) lifestyle characteristics 45 of the user in response to a lifestyle characteristics enquiry 44. The touch panel 2a is used to present questions 60 in one or more cognitive ability tests (cognitive tests) and to input cognitive ability data 61, including answers to the questions of the cognitive ability test, into the presentation system 10. In addition, the touch panel 2a displays the report 50 which includes a presentation of the dementia risk.

The user terminals used for input/output of such information are not limited to tablets 2 with the touch panel 2a. The user terminals may have any configuration capable of recognizing the individual characteristics of the user for whom the dementia risk is to be determined and capable of presenting the dementia risk. As examples, a personal computer or a smartphone may be used in place of a tablet. Also, in place of a touch panel that is an input/output unit where the output unit and the input unit are integrated, a configuration with a separate output unit and input unit may be used. As specific examples, a keyboard or a microphone may be used as the input unit and a display, a speaker or a printing device may be used as the output unit.

The input unit and the output unit of the user terminal do not need to be provided in the same device, and do not need to be disposed at positions close to each other, such as in a room. As one example, a display, a speaker, a printing device, or the like as the output unit of a user terminal may be disposed in a medical workstation provided in a different place to the location where the MRI apparatus or the like has been installed.

The server 7 that operates as the presentation system 10 includes a memory (data storage unit or data storage region) 16 and a processor (CPU) 8 that operates as various functions (functional units) by loading a program (program product) 19 stored in the memory 16. The memory 16 includes a database (first database) 17 including sample brain health data (sets of sampled brain health data) 17a of sample subjects (sampled subjects, sampling participants, sampled targets) including at least one type of data for sample brain state data (sets of sampled brain state data) 17d, which are data relating to states of the brains of sample subjects, and sample cognition data (sets of sampled cognition data, cognitive ability data) 17e, which are data relating to cognitive abilities as functions of the brains of the sample subjects, and the user library 18 in which the brain health data (set or sets of brain health data) 18a of the user (that are the user brain health data) are stored.

The database 17 further includes individual characteristics 17b containing at least one of the age, gender, and physical information of the sample subjects. The sample brain health data 17a are stored in association with respective individual characteristics 17b. The database 17 further includes lifestyle characteristics 17c including at least one of lifestyle habits and the living environment of the sample subjects. The sample brain health data 17a are also stored in association with respective lifestyle characteristics 17c.

Here, the expression "sample subjects (sampled targets)" includes not only people who have developed dementia (including mild dementia) and people who are suspected of having developed dementia, but also healthy people who have not developed dementia. The individual characteristics 17b include at least one of age, gender and physical information. The "physical information" included in the individual characteristics 17b refers to physical information that can be expressed quantitatively. Specific examples include such as but not limited to, height, weight, body fat percentage, BMI, and cholesterol level.

The lifestyle characteristics 17c include information relating to at least lifestyle habits and/or living environment. The expression "lifestyle habits" here refers to habitual behavior in daily life that has an effect of increasing or decreasing dementia risk. Specific examples include such as, but not limited to, the amount of exercise, the amount and types of food consumed, the length and quality of sleep, the amount and frequency of communication, the amount and frequency of hobby activities, the amount and frequency of alcohol consumption, and the amount and frequency of smoking. The expression "living environment" refers to environmental factors in daily life that have an effect of increasing or decreasing dementia risk. Specific examples include such as, but not limited to, where the user lives (living environment), occupation, educational background, annual income, and number of household members.

In the database 17, the sample brain health data (multiple sets of sampled brain health data) 17a of the sample subjects, which include the sets of data (sample brain state data) 17d pertaining to the respective states of the brain of the sample subjects and the sets of data (sample cognition data) 17e pertaining to the results of cognitive ability tests performed on the respective sample subjects, are stored in a format where the sample brain health data 17a of the sample subjects are associated with the individual characteristics 17b and the lifestyle characteristics 17c of the respective sample subjects. The user library 18 stores, in addition to the latest (present) version of a set of the user brain health data 18a which includes a set of data (user brain state data) 41 relating to the state of the brain of the user and a set of data (user cognition data) 61 relating to the cognitive ability of the user, one or more sets of user brain health data that were acquired in the past. The user library 18 may also store an individual characteristic (user individual characteristic) 43 and a lifestyle characteristic (user lifestyle characteristic") 45 of each user. The presentation system 10 compares the data (user brain health data) 18a relating to the health state of the brain of the user (target user) whose risk is to be determined and the multiple sets of the data (sample brain state data) 17a relating to the brain states of the sample subjects to recognize the dementia risk of the target user.

Here, the data 17a and data 41 both relating to the brain state include the form of quantitative parameters based on the states of the brains that affect cognitive abilities and are the directly measured or observed data of the brains. That is, this dementia risk presentation system 10 uses such directly measured data as the data relating to the state of the brain, instead of the indirect data showing brain function estimated from brain waves, movements, and the like of the user used in a conventional system. By doing so, this presentation system 10 is not affected by the estimation accuracy regarding brain function when recognizing dementia risk. As a result, by using the presentation system 10, dementia risk can be recognized more accurately than with the conventional system.

The presentation system 10 includes a first recognition unit 11 that acquires (recognizes) the user brain health data 18a. The user brain health data 18a includes at least one of type of data for the user brain state data 41 that is data relating to the state of the user's brain and the user cognition data 61 that is data relating to cognitive ability, which is a function of the brain. The first recognition unit 11 includes an input unit (input function, input interface, brain state input) 14 for acquiring the user brain state data 41 and an input unit (input function, input interface, cognitive ability input) 15 for acquiring the user cognition data 61. The cognition data input unit 15 sends a cognitive ability test 60 to the user terminal 2 and recognizes the user cognition data 61 based on answers to the test 60. The cognition data input unit 15 is not limited to inputting of direct information from the terminal 2 and may indirectly acquire information relating to the user through input via a paper medium or information provided via a doctor or other specialist. The same methods may apply to the recognition (acquisition, input) of other information.

One example of the cognitive test 60 is evaluation of the function of each of the five cognitive domains. The cognitive domains include short-term memory, working memory, executive function, spatial cognition, and calculation. Short-term memory indicates the ability to retain and recall what the subject has learned for a short period and is said to involve the hippocampus of the brain. Working memory is the ability to manipulate retained memories in different ways, such as "remembering a phone number and pressing buttons for that number to make a call", and is said to involve the prefrontal cortex and temporoparietal part of the brain. Executive function is the ability to maintain and update rules and to control behavior and thoughts, such as a "procedure" and "changing ingredients according to the situation" when cooking, and is said to particularly involve the prefrontal cortex of the brain. Spatial cognition is the ability to instantly grasp and understand which direction a figure or object is facing in space and is said to involve the parietal lobe of the brain. Calculation is the ability to perform the four arithmetic operations and is said to involve various regions of the brain, but in particular the temporoparietal part of the brain.

The cognition data input unit 15 performs the cognitive ability test 60 and acquires evaluations for each of these cognitive ability domains as the user cognition data 61. By performing statistical processing as described later on the evaluation for each cognitive ability domain, the cognition data 61 can be used as an index for accurately determining the function of the brain.

The presentation system 10 further includes a second recognition unit (individual characteristics input unit or second input interface) 12 that recognizes (acquires) a user individual characteristic 43, which is the individual characteristic of each user, and a third recognition unit (lifestyle characteristics input unit or third input interface) 13 that recognizes (acquires) a user lifestyle characteristic 45, which is the lifestyle characteristic of each user. The individual characteristics input unit 12 may send a questionnaire (survey) 42 relating to individual characteristics to the user terminal 2 and recognize the individual characteristic 43 of each user from the answers to the questionnaire 42. When an electronic medical record or the like relating to the user has been created in advance, the individual characteristics input unit 12 may recognize the user individual characteristics 43 based on the content of this electronic medical record or the like.

The lifestyle characteristics input unit 13 may send a questionnaire (survey) 44 relating to lifestyle characteristics to the user terminal 2 and recognize the lifestyle characteristic 45 of each user from the answers to the questionnaire 44. When an electronic medical record or the like relating to the user has been created in advance, the lifestyle characteristics input unit 13 may recognize the user lifestyle characteristics 45 based on the content of this electronic medical record or the like.

The presentation system 10 also includes a recognition/prediction unit (prediction generator, prediction unit or prediction function) 20, which recognizes the present dementia risk and predicts the future risk for each user, and a risk presentation unit (risk presentation device, risk presenter, risk information provider, or risk presentation function) 30, which presents or provides information including the dementia risk to the user. The recognition/prediction unit 20 includes a present risk recognition unit (present risk recognizer) 21 that recognizes the present risk, a prediction unit (predictor) 22 that predicts the future risk, and a score calculation unit (score calculator) 23 that converts the risk into a score.

The present risk recognition unit 21 recognizes a chronological transition line (an aging line), which indicates transitions in the user brain health data 18a of the user from the past to the present based on the past and present user brain health data 18a, and also recognizes a risk line for dementia which is determined by referring to age-related transitions in the sample brain health data 17a associated with individual characteristics 17b that correspond to the user individual characteristic 43. The present risk recognition unit 21 outputs the chronological transition line and the risk line of the user via the risk presentation unit 30 to the user terminal 2.

This risk recognition unit 21 acquires the sample brain state data 17d and the sample cognition data 17e associated with the individual characteristics 17b corresponding to the user individual characteristic 43 from the database 17. The acquired sample brain state data 17d are then compared with the user brain state data 41, and the acquired sample cognition data 17e are compared with the user cognition data 61. After doing so, the dementia risk of the user may be recognized based on the result of these comparisons. As described earlier, the sample brain state data 17d and the user brain state data 41 are not limited to the volumes of the hippocampus, and may be the volumes of the entire brains or may be the volumes of at least one predetermined region of the brains.

The prediction unit 22 predicts a chronological prediction line (an aging prediction line) indicating transitions in the user brain health data that have been predicted for the future from the user's present state based on his chronological prediction line, and enables the chronological prediction line (aging prediction line) to be displayed along with the chronological transition line and the risk line via the risk presentation unit 30. Based on the results of a comparison between user brain health data 18a for the present or predicted for the future and the sample brain health data 17a associated with the individual characteristics 17b corresponding to user individual characteristic 43, the score calculation unit 23 calculates a score indicating the dementia risk of the user, or alternatively a symbol corresponding to the score, and enables the score or symbol to be presented via the risk presentation unit 30.

The risk presentation unit 30 includes a chart display unit (chart display interface) 31 that displays charts or graphics for the chronological transition line, the chronological prediction line, the risk line, and the like, and a score presentation unit (score presentation interface) 32 that displays the calculated score. The chart display unit 31 includes a prediction presentation unit 31a, which displays predictions including the chronological prediction line, and a comparison presentation unit 31b, which displays the results of simulating a plurality of cases in which the lifestyle characteristics are changed. The score presentation unit 32 includes a first score presentation unit 32a that displays the score (or a corresponding symbol or the like) for the present risk, a second score presentation unit 32b that displays a score or the like for the predicted risk, a third score presentation unit 32c that displays a score or the like for a predicted risk using lifestyle characteristics as factors, and a fourth score presentation unit 32d that displays a score or the like for a risk produced by simulating cases with changed lifestyle characteristics.

Fig. 2 depicts one example of a report 50 provided to the user by the presentation system 10. This report 50 may be a screen or digital data presented to the user terminal 2 when the dementia risk has been determined, may be an audio file, or may be printed matter, a booklet, or the like provided to the user via the user terminal 2 or alternatively through the mail or the like. The report 50 includes a brain image display area (brain image output field) 59, a chronological transition display area (aging display area, chronological information output filed) 51, a score display area (score output filed) 57, and a lifestyle characteristics display area (lifestyle input/output filed) 58, with information on dementia risk or user lifestyle characteristics being presented in each of these areas.

In more detail, images of the user's brain included in the user brain state data 41 acquired by the MRI 3a are displayed in the brain image display area 59. Graphs (or charts) are displayed via the presentation unit 30 in the chronological information display area 51 as the information for the dementia risk recognized by the risk recognition/prediction unit 20. These graphs indicate the result of comparing the sample brain state data and the user brain state data. The graphs include the chronological transition line (aging line) 52, the chronological prediction line (aging prediction line) 53, and the risk line 54.

In the score display area 57, a rank 55 relating to an overall risk judgment indicated using the letters A to D and F, a brain age 56a indicated as a number, and an arrow 56b which is a symbol relating to a long-term prediction are displayed as the dementia risk recognized by the risk recognition/prediction unit 20. In addition, a legend 56c relating to the rank 55 is displayed in the score display area 57. The value of the rank 55, the value of the brain age 56a, and the slope of the arrow 56b are determined according to scores obtained based on the result of the comparison between the sample brain state data 17d and the user brain state data 41 and the result of the comparison between the sample cognitive ability data 17e and the user cognitive ability data 61. Rank A indicates that the brain function (the working of the brain) is high, rank B indicates that the brain function is normal, rank C indicates that the brain function is low, and rank D indicates that the brain function has decreased to a level worthy of caution. Rank F indicates that treatment has already commenced in response to a fall in brain function.

The user lifestyle characteristics 45 are displayed in the lifestyle characteristics display area 58. The results of any changes made via the touch panel 2a are reflected in the user lifestyle characteristics 45.

Note that this is merely one example of a method of presenting the dementia risk in this type of display format. In other words, the method of presenting the dementia risk using the presentation system 10 is not limited to this configuration, and any configuration that presents the result of a comparison between the user brain health data 18a and the sample brain health data 17a associated with the individual characteristics and/or lifestyle characteristics of the user may be used. As one example, the layout of the screen (report) 50 may be changed as appropriate. It is also possible to display only a graph or only a score. Additionally, out of the displayed information relating to scores, only one or two out of the rank, the brain age, and the arrow may be displayed. Information may be presented in a completely different format to the displays described above. As one example, an image of the user's brain and an image of the brain of a sample subject who has the same individual characteristics as the user and is in an ideal health state may be displayed side by side. It is also possible to present results using audio.

The content displayed in the chronological information display area 51 will now be described further. In this report 50, graphs that have the hippocampal volume on the vertical axis and age on the horizontal axis are displayed for showing the dementia risk in the chronological information display area 51. The graphs include the chronological transition line (chronological result line) 52, the chronological prediction line 53, and the risk line 54. The chronological transition line 52 indicates transitions in the user brain state data 41 for the user from the past to the present. The chronological prediction line 53 indicates transitions for the user brain state data 41 of the user that have been predicted from the present to the future. The risk line 54 indicates the degree of dementia risk that the user and others should be wary of, and divides the area of the graph into a normal range 54a and a zone of caution needed (danger zone) 54b.

The user and others compares the slope of the chronological transition line 52 and the slope of the chronological prediction line 53 with the slope of risk line 54 and/or refers to the position of the chronological transition line 52 and the position of the chronological prediction line 53 (in more detail, whether the positions are within the range of the normal range 54a or the range of the caution zone 54b) to grasp the magnitude of the user's dementia risk and the trend in the dementia risk.

During the process of creating the chronological transition line 52 and the chronological prediction line 53, the user brain state recognition unit 14 first recognizes the user's present and past brain state data 41. In more detail, the user brain state recognition unit 14 uses a predetermined algorithm to calculate the hippocampal volume from cross-sectional images of the user's brain acquired using the MRI 3a for the past (in the present embodiment, when the user was 41 years old) and the present (when the dementia risk is being determined, in the present embodiment when the user is 45 years old). As the predetermined algorithm, it is possible to use a technique that predicts the hippocampal volume using a generation model for transitions over time in images using a neural network. To further improve accuracy, biomarker data information that is highly correlated with lifestyle observations may be added.

The present risk recognition unit 21 of the risk recognition/prediction unit 20 recognizes the chronological transition line 52 relating to the brain state of the user based on the user brain states 41 recognized by the user brain state recognition unit 14.

In more detail, as depicted in Fig. 3, the present risk recognition unit 21 first plots the hippocampal volume of the user in the past (at 41 years old) and the hippocampal volume of the user in the present (at 45 years old) on a graph where the vertical axis represents the hippocampal volume and the horizontal axis represents age. After that, the risk recognition unit 21 generates the chronological transition line 52 by connecting the plotted volumes with a line segment. Based on the user individual characteristic 43, the risk recognition unit 21 acquires sample brain state data 17d associated with the individual characteristics 17b that correspond to the user individual characteristic 43 from the database 17.

One example user has "male" as an individual characteristic 43. The risk recognition unit 21 acquires sample brain state data (that is, hippocampal volume) 17d associated with "male" as the individual characteristics 17b. The risk recognition unit 21 then determines the risk line 54 with reference to transitions over time in the sample brain state data (multiple sets of the sample brain state data) 17d that have been recognized based on this individual characteristic.

In Fig. 3, the vertical axis is hippocampal volume, the horizontal axis is age, and multiple sets of the sample brain state data 17d that have been extracted according to the individual characteristics 17b are plotted. The black circles and white circles in Fig. 3 represent individual datapoints of the multiple sets of the sample brain state data 17d, with the white circles representing the sets of the sample brain state data 17d belonging to a group with high alcohol consumption and the black circles representing the sets of the sample brain state data 17d of others. In the graph, a first mean line 66 indicating the mean hippocampal volume of the extracted sample brain state data 17d including the group with high alcohol consumption has been drawn using a solid line, and a second mean line 67 indicating the mean hippocampal volume of the sample brain state data 17d for only the group with high alcohol consumption has been drawn using a broken line. This statistical processing may be performed in advance or may be performed by the risk recognition unit 21.

The risk recognition unit 21 generates a risk line 54 as shown using a dotted line at a position below the first mean line 66. In more detail, a line is generated based on values $(x1-2\sigma)$ that are $2\sigma$ (where "$\sigma$" represents the standard deviation of the hippocampal volume (the sample brain state data) at each age) lower than the respective values $x1$ of the first mean line 66, in this case, that is the line indicating 2.3% from the bottom, and this generated line is recognized as the risk line 54.

Note that the method of recognizing the risk line 54 according to the present invention is not limited to the above method and it is possible to use any method that decides the line by referring to age-related transitions in the multiple sets of the sample brain state data, especially based on the plurality of sets of the sample brain state data 17d associated with individual characteristics corresponding to the user individual characteristics or characteristic. As one example, when a user individual characteristic is "male" as in the present embodiment, the risk line 54 may be determined based on the mean line of the plurality sets of the sample brain state data 17d associated with the different or limited lifestyle characteristics 17c corresponding to the user lifestyle characteristics 45 out of the multiple sets of the sample brain state data 17d, not just all of the multiple sets of the sample brain state data 17d associated with the individual characteristic "male". In more detail, the risk line 54 may be determined based on the second mean line 67, which is a trend line for only the group with high alcohol consumption.

Also, as one example, a mean line itself may be used as the risk line 54 in place of a line based on values given by adding $(-2\sigma)$ to each value of the mean line. Alternatively, the risk line 54 may be generated by using different values (as one example, values given by adding $-3\sigma$ to the respective values of the mean line). The risk line 54 may also be generated by referring to simply the order of data or the like, without the risk line 54 being based on data that have been statistically processed as described above. In more detail, it is possible to generate the risk line 54 with the sets of data with a predetermined rank (for example, the lowest three sets of the data from the bottom) out of the sets of the sample brain state data 17d associated with individual characteristics corresponding to the user individual characteristic.

Next, the prediction unit 22 of the risk recognition/prediction unit 20 predicts (forecasts, calculates) the chronological prediction line (aging prediction line) 53 indicating transitions of the user brain health data predicted for the user from the present to the future based on the chronological transition line 52. The prediction unit 22 includes a function that predicts the chronological prediction line 53 based on sample brain health data 17a, which is associated with the individual characteristics 17b corresponding to the user individual characteristic 43, the lifestyle characteristics 17c corresponding to the user lifestyle characteristic 45, and age, and also on the chronological transition line 52.

Based on the user lifestyle characteristics 45, the prediction unit 22 generates the chronological prediction line 53 based on the sets of the sample brain state data 17d associated with the lifestyle characteristics 17c corresponding to the user lifestyle characteristic 45, and in the present embodiment, the second mean line 67 of the sets of the sample brain state data (white circles) 17d for the user's individual characteristic of having high alcohol consumption. In more detail, the prediction unit 22 refers to transitions in the second mean line 67 from the base age (starting age) when the user brain state data 41 is taken (45 years old, which is the age at the most recent time in the present embodiment) to a predetermined age (in the present embodiment, 50 years old) and generates, as the chronological prediction line 53, a line that traces the same transitions as the second mean line 67, starting from the base age on the chronological transition line 52.

Note that in the present embodiment, the user lifestyle characteristics 45 are referred to when recognizing the chronological prediction line 53. This is performed to recognize an chronological prediction line (and in turn a dementia risk trend) that is highly reliable. However, the method of recognizing the chronological prediction line in the present invention is not limited to this method, and any method that performs recognition based on the chronological transition line 52 may be used. As one example, the slope of the chronological transition line 52 may be recognized, and then used to generate and recognize the chronological prediction line 53. Any method that determines the line by referring to transitions relating to age in the sample brain state data 17d based on the sample brain state data 17d associated with the lifestyle characteristics 17c corresponding to the user lifestyle characteristics 45 may be used. As one example, it is possible to refer not only to the sample brain state data 17d relating to a single lifestyle characteristic 17c, but also to the sample brain state data 17d relating to two or more lifestyle characteristics 17c.

A regression equation for an evaluation factor x composed of at least one of the three factors "sample brain state data", "sample cognition data", and "user individual characteristics" for an unspecified number of sample subjects of the age z is expressed by Equation (1) below. When a regression equation for the evaluation factor x for sample subjects with a predetermined lifestyle characteristic (for example, high alcohol consumption) out of an unspecified large number of sample subjects of age z is expressed by Equation (2) below, an evaluation factor $x_{t+1}$ for the next time point t+1 (where t represents a discrete period such as 5 years or 10 years) is predicted based on the evaluation factor $x_t$ at the present time point t according to Equation (3) below.

$$x=\beta_0+\beta_1 z \text{ (where } \beta_0, \beta_1 \text{ are regression coefficients) ... (1)}$$

$$x'=\beta_0'+\beta_1' z \text{ (where } \beta_0', \beta_1' \text{ are regression coefficients) ... (2)}$$

$$x_{t+1}=x_t+\{\text{IF lifestyle characteristic is present, THEN}(x_{t+1}'-x_t')$$
$$\text{ELSE}(x_{t+1}-x_t)\} \text{ ... (3)}$$

The prediction presentation unit 31a, which displays one or more charts, in the risk presentation unit 30 displays the chronological transition line 52, the chronological prediction line 53, and the risk line 54 side by side so as to allow comparison in the chronological information display area 51 of the report 50. Although it is also possible for the prediction presentation unit 31a to display a plot indicating the multiple sets of the sample brain state data 17d, the first mean line 66, and the second mean line 67, these have been deleted to make it easier for the user and others to see the lines 52 to 54. The prediction presentation unit 31a may display or output the normal range 54a above and the caution zone 54b below the risk line 54 using different colors. The prediction presentation unit 31a also plots the ages (in the present embodiment, 41 years, 45 years, and 50 years) used as standards for the chronological transition line 52 and the chronological prediction line 53 on the horizontal axis of the graph.

In the present embodiment, when the chronological prediction line 53 is above the risk line 54, this means that the value $cdf(x_{t+1}|\mu_{t+1}, \sigma_{t+1})$ at a future time point t+1 for the cumulative density function $cdf(x|\mu, \sigma)$ of the evaluation factor x expressed by Equation (4) below is greater than $2\sigma$.

$$cdf(x|\mu, \sigma) = (1/2) \{1+erf(x-\mu)/(2\sigma^2)^{1/2}\})$$
$$\text{(where erf is the error function) ... (4)}$$

The prediction unit 22 may show or display the chronological prediction line 53 with an estimation width 53w indicating a region with high probability. As depicted in Fig. 4, the first mean line 66 and the second mean line 67 both include an error, and their 95% CI (Confidence Intervals) are respectively indicated by the lines 66a and 67a. The prediction unit 22 may calculate the estimation width 53w of the chronological prediction line 53 for the latest time point t for each user from the estimation width of the second mean line 67 of the same age at the future time point t+1, and the prediction presentation unit 31a may display the estimation width 53w in the chronological information display area 51.

Fig. 5 depicts a different example of where the chronological prediction line 53 is indicated using an estimated width 53w. In this example, there is the suggestion that the estimation width 53w of the dementia risk may fall below the risk line 54 from pre-MCI (that is, a stage before Mild Cognitive Impairment) to MCI at a future time point t+1, indicating a probabilistic risk of developing AD (Alzheimer's Disease or Alzheimer-type dementia).

In this way, the presentation system 10 presents the dementia risk in a format with lines following the user's age, such as the chronological transition line 52, the chronological prediction line 53, and the risk line 54 that can be compared. By doing so, it is possible for the user and others to grasp not only the scale of the dementia risk for the user but simultaneously also the trend in the dementia risk of the user (such as whether the risk is worsening, improving, or deterioration is being suppressed).

The prediction unit 22 of the presentation system 10 also includes a comparison/prediction unit 22a that recognizes a first chronological prediction line 53 based on a first user lifestyle characteristic 45 and a second chronological prediction line 53a based on a second user lifestyle characteristic 45 for the identical user (same user). When the user lifestyle characteristics 45 are changed by the user and others, the displayed content relating to the dementia risk will also vary according to the changed characteristics. For this reason, the presentation system 10 includes a comparison/prediction unit 22a that simulates results for when the user lifestyle characteristics 45 have been changed, and a comparison presentation unit 31b that presents the results side by side or consecutively to allow comparison.

Fig. 6 depicts two types of report 50 in which the chronological prediction line 53 has changed due to a change in the user lifestyle characteristic 45. In the report 50 depicted in Fig. 6 (a), the level of alcohol consumption indicated in the lifestyle characteristics display area 58 is set at "daily", and in the report 50 depicted in Fig. 6 (b), the level of alcohol consumption indicated in the lifestyle characteristics display area 58 has been changed to "two or three times a week". As a result, in the report 50 depicted in Fig. 6(b), the chronological prediction line 53a displayed in the chronological information display area 51 is located above the chronological prediction line 53 depicted in the report 50 in Fig. 6(a) and has a slope that moves away from the risk line 54, indicating that the dementia risk decreases.

In the comparison/prediction unit 22a, when the level of alcohol consumption in the user lifestyle characteristics 45 is changed from "daily" to "two to three times a week", a new mean line (or "third mean line", not illustrated) is calculated by further extracting samples relating to the alcohol consumption is low out of sets of the sample brain state data 17d extracted in Fig. 3, and a new chronological prediction line 53a is generated based on this mean line using the same processing as the chronological prediction line 53. The comparison presentation unit 31b may present the first chronological prediction line 53 for a previous condition and the second chronological prediction line 53a for a subsequent (pseudo) condition as separate reports 50 or may present the prediction lines side by side in the same report 50. The comparison presentation unit 31b may consecutively display the first chronological prediction line 53 and the second chronological prediction line 53a so as to allow comparison. In more detail, after a predetermined time has elapsed and the first chronological prediction line 53 is no longer displayed, the second chronological prediction line 53a may be displayed within a period where the user and others can still recall the rough shape of the first chronological prediction line 53.

The comparison/prediction unit 22a and the comparison presentation unit 31b may obtain and present the chronological prediction line 53 by changing the conditions of the user lifestyle characteristics 45 any number of times without being limited to changing the conditions twice. Thanks to this function of the presentation system 10, it is possible for the user and others to intuitively grasp how the dementia risk varies according to variations in the lifestyle characteristics 45. By doing so, the user and others can grasp lifestyle characteristics that are to be improved in specific terms, and an incentive is provided to improve the lifestyle characteristics.

The risk recognition/prediction unit 20 of the presentation system 10 includes the score calculation unit 23 that converts the dementia risk into a score, and the risk presentation unit 30 includes a score presentation unit 32 that presents the dementia risk based on the score. The score calculation unit 23 compares the user brain state data 41 and the user cognition data 61, with the sample brain state data 17d and the sample cognitive ability data 17e that have been recognized based on the user individual characteristic 43 and the user lifestyle characteristic 45, assigns a score based on the result of this comparison, and determines a rank, brain age, and corresponding symbol based on this score.

As one example, the value cdf $(x_t | \mu_t, \sigma_t)$ at the present time t of the cumulative density function cdf $(x|\mu, a)$ for the evaluation factor x indicated in Equation (4) above in the sample brain state data 17d and the like may be defined as the score "S". The rank 55 may be decided according to which of a plurality of numerical ranges (as examples, the four numerical ranges $0.0 < S \leq 0.25$, $0.25 < S \leq 0.50$, $0.50 < S \leq 0.75$, and $0.75 < S \leq 1.0$) includes the score S. The score calculation unit 23 may also include a function 23a that decides the brain age 56a by referring to a table in which various values of the score S or a plurality of numerical ranges are associated with a plurality of brain ages.

The score calculation unit 23 may also include a function 23b that determines the symbol 56b corresponding to the score based on the deviation of the value cdf $(x_{t+1} | \mu_{t+1}, \sigma_{t+1})$ at the future time point t+1 of the cumulative density function cdf $(x|\mu, \sigma)$ for the evaluation factor x from the value cdf $(x_t | \mu_t, \sigma t)$ at the present time t. When the deviation is a positive value or is greater than or equal to a predetermined positive value, it is determined that there is the possibility of the risk falling, when the deviation is a negative value or is equal to or less than a predetermined negative value, it is determined that there is the possibility of the risk rising. When the deviation is 0 or the value is less than a predetermined positive value and larger than a predetermined negative value, it is determined that there is no change in the risk. The symbol 56b corresponding to the score S is then determined according to whether the risk is "possible fall", "possible rise", or "no change".

The function 23b that decides the symbol may include a function of determining the long-term prediction symbol 56b corresponding to the score S based on whether the value cdf $(x_{t+1} | \mu_{t+1}, \sigma_{t+1})$ of the evaluation factor x at the future time point t+1 is 0.023 (0.023 is equivalent to (-2σ, (that is, the threshold indicating the lowest 2.3%)) of the population) or higher, or is below 0.023.

Note that the method of calculating the score is not limited to a method that uses parametric modeling, such as the method that uses the cumulative density function described above. As one example, a method using non-parametric ranking may be used. In more detail, various methods for calculating quantiles using mean rank, median rank, approximation, and the like may be used.

The score presentation unit 32 of the presentation system 10 displays (outputs) the rank 55 decided based on the score, the brain age 56a, the arrow 56b, and the legend 56c relating to ranks on the touch panel 2a of the tablet 2 or in the report 50. In this way, the presentation system 10 presents the dementia risk as a score or a symbol corresponding to a score. By doing so, the user and others can intuitively grasp how high or low the dementia risk for the user is.

The score calculation unit 23 may include, in addition to the function of calculating the score of the present dementia risk and displaying the score via the first score presentation unit 32a, a function 23a that calculates the score of the dementia risk for the user in the future, as one example, one year later. The function 23c that calculates the future score may calculate a score indicating the future dementia risk of the user based on the result of a comparison between the user brain health data 18a of a first age on the chronological prediction line 53 and the sample brain health data 17a associated with individual characteristics corresponding to individual characteristics 17b obtained by including the first age along with the user individual characteristic 43. The function 23c may output, by using the second score presentation unit 32b, the calculated score or a symbol corresponding to the score together with or in place of the chronological prediction line 53.

The function 23c that calculates the future score may calculate the score indicating the future dementia risk of the user based on the result of a comparison between the user brain health data 18a of a first age on the chronological prediction line 53 and the sample brain health data 17a associated with individual characteristics 17b corresponding to individual characteristic obtained by including the first age along with the user individual characteristic 43 and the lifestyle characteristics 17c corresponding to the user lifestyle characteristic 45. The function 23c may output, by using the third score presentation unit 32c, the calculated score or a symbol corresponding to the score together with or in place of the chronological prediction line 53. The function 23c for calculating the future score may include a function for calculating the score based on the user brain health data 18a that has been specified by the user or that has been arbitrarily selected, in place of the user brain health data 18a on the chronological prediction line 53.

The function 23c that calculates the future score may include a simulation function (simulator) 23d that changes the user lifestyle characteristics 45 and confirms how the score changes. This function 23d calculates a first score that indicates the future dementia risk of the user based on the result of a comparison between the user brain health data 18a of the first age on the first chronological prediction line 53 that reflects the first user lifestyle characteristic 45 and the sample brain health data 17a associated with the individual characteristics 17b corresponding to individual characteristic obtained by including the first age along with the user individual characteristic 43 and the lifestyle characteristics 17c corresponding to the first user lifestyle characteristic 45. In addition, this function 23d may calculate a second score that indicates the future dementia risk of the user based on the result of a comparison between the user brain health data 18a of the first age on the second chronological prediction line 53a that is based on the second user lifestyle characteristic 45 and the sample brain health data 17a, which is associated with individual characteristics 17b corresponding to individual characteristic 43 obtained by including the first age along with the user individual characteristic and the lifestyle characteristics 17c corresponding to the second user lifestyle characteristic 45. The fourth score presentation unit 32d may output the first and second scores, or symbols corresponding to the first and second scores, together with or in place of the chronological prediction line. When the lifestyle characteristics of the user are changed by the user and others, the displayed contents relating to the dementia risk also vary according to the changed conditions.

In the report 50 depicted in Fig. 7(a), the condition of the alcohol consumption in the lifestyle characteristics display area 58 is "daily", and in the report 50 depicted in Fig. 7(b), the condition of the alcohol consumption is changed to "two or three times a week". Each report 50 displays the dementia risk at a point in the future, for example, one year later, as a score, and by changing the amount of alcohol consumption from "daily" to "two or three times a week", the chronological prediction line 53 changes, and the sets of the sample brain health data 17a used in comparisons are changed from the "high alcohol consumption group" to the "low alcohol consumption group". In this case, the overall risk determination (overall risk assessment) 55 is changed from "B" to "A", the brain age 56a becomes younger, and the long-term prediction symbol 56b also changes to become flat.

In this way, in the presentation system 10, the future dementia risk can be outputted as a score, a rank, or the like in accordance with changes to the user lifestyle characteristics 45. This means the user and others can intuitively grasp variations in the dementia risk in keeping with variations in lifestyle characteristics. By doing so, since the user and others can grasp the lifestyle characteristics that should be improved in specific terms, it is possible to provide an incentive for improving the lifestyle characteristics.

Fig. 8 depicts an example of a different type of report indicating the dementia risk. This report 50 is outputted from the risk presentation unit 30 of the presentation system 10 in the same manner as above. The report 50 includes, in addition to the score display area 57 and the chronological information display area 51 for the user brain state data 41, an analysis result display area 75 for the user cognitive ability data 61 and a chronological information display area 71 for the user cognitive ability data 61. The score display area 57 includes, in addition to the rank 55 and the brain age 56a, a display of a CQ score (Cognitive Quotient score) 76. The CQ score 76 is a comprehensive cognitive ability and brain function score of an individual based on various cognitive function tests, and if the average is set at 100 and the standard deviation is set at 15, is a scale that is compatible with scoring of IQ (intelligence quotient) and other cognitive tests. The CQ score (cq) 76 is calculated according to Equation (5) below.

$$cq = \sum_{i \in A} z(x_i, \mu_i, \sigma_i)$$

$$z(x, \mu, \sigma) = \frac{x - \mu}{\sigma} \quad \cdots \quad (5)$$

The variable $x_i$ is the evaluation result of the respective domains in the cognitive ability test 60, and for this report 50, is the test result for the functions of short-term memory, working memory, executive function, spatial cognition, and calculation. The test items (domains) included in the cognitive test 60 are not limited to these, and may be a combination of forward counting, backward counting, calculations, Stroop tests, mental rotations, or combinations of other test items. In Equation (5), the total score (cq or CQ score) 76 is the sum of the scores of each test item (domain) after standardizing (z) to a normal distribution.

In this report 50, the cumulative density cdf (cq|μ, σ) 77 for the same age is calculated using the cumulative density function indicated in Equation (4), and the rank 55 is found according to the numerical range that includes the calculated CQ score 76. As one example, when a normal distribution with a mean of 100 and a standard deviation of 15 is assumed, the region "Above" where the deviation value (standard score) exceeds 110 is set as "rank A", the region "Average and Low Average" where the deviation value is 80 to 110 is set as "rank B", the region "Low" where the deviation value is 70 to 79 is set as "rank C", and the "Very Low" region where the deviation value is below 70 is set as "rank D".

Note that the cognitive ability tests are not limited to the above examples, and a score may be evaluated according to the tests or investigations that measure the health states of the brain listed in Figs. 9 and 10 (including the state of cognitive function and the presence and degree of brain disease and psychiatric disorders) or another test or investigation of a similar type.

The analysis result display area 75 in the report 50 includes a radar chart 78 indicating the results of each item (that is, short-term memory, working memory, executive function, spatial cognition, and calculation) in the cognitive ability test 60 and standard values for the same age of the user. The chronological information display area 71 of the user cognitive ability data 61 indicates the chronological transition lines 72a to 72e for the respective items (short-term memory, working memory, executive function, spatial cognition, and calculation) in the user cognitive ability data 61 and standard lines 74a to 74e of the age group of the user as risk lines.

In the chronological information display area 51 of the report 50, in addition to the chronological transition line 52 of the user brain state data 41 and the risk line 54, a mean transition line 65 for the same age group is indicated based on the user individual characteristics 43. From the displayed information, the user can clearly grasp the dementia risk and the brain health over time by looking at the report 50.

Fig. 11 depicts an overview of the processing in the presentation system 10 described above by way of a flowchart. In step 101, the first recognition unit 11 recognizes (acquires) the user brain health data 18a including at least one type of data for the user brain state data 41, which is data relating to the state of the brain of the user, and the user cognition data 61 of the user, which is data relating to cognitive ability, one function of the brain. In step 102, the second recognition unit (or "individual characteristic input unit") 12 recognizes (acquires) the user individual characteristic 43, which is the individual characteristic of the user. In step 103, the presentation system 10 determines whether prediction processing is required, and if prediction processing is not required, in step 104, the risk presentation unit 30 presents the risk by way of the chronological transition line 52, which indicates transitions in the user brain health data 18a for the user from the past to the present based on past and present user brain health data 18a, and the risk line 54, which relates to dementia and is determined by referring to the age-related transitions in the sample brain health data based on the sample brain health data 17a associated with the individual characteristics 17b corresponding to the user individual characteristic 43.

In step 105, the risk presentation unit 30 further presents the score 55, which indicates the dementia risk of the user, or a symbol according to the score based on the result of a comparison between the user brain health data 18a and the sample brain health data 17a associated with the individual characteristics 17b corresponding to the user individual characteristic 43. Then, in step 106, the risk presentation unit 30 generates a report 50 including the information above and provides the report 50 to the user. The report 50 at this stage is a report that does not include an chronological prediction line.

When prediction processing is required in step 103, the presentation system 10 further determines in step 107 whether the user lifestyle characteristic has been inputted. If the user lifestyle characteristic 45 has been inputted, in step 108, the third recognition unit (lifestyle characteristics input unit) 13 recognizes (acquires) the user lifestyle characteristic 45, which is the lifestyle characteristic of the user. In step 109, the prediction unit 22 of the risk recognition/prediction unit 20 predicts (calculates) the chronological prediction line 53 based on the chronological transition line 52 and the sample brain health data 17a, which are associated with the individual characteristics 17b corresponding to the user individual characteristic 43, the lifestyle characteristics 17c corresponding to the user lifestyle characteristic 45, and the age. In step 108, when the user lifestyle characteristic 45 has not been inputted, in step 109, the prediction unit 22 generates the chronological prediction line 53 based on the chronological transition line 52 and the sample brain health data 17a associated with the individual characteristics 17b.

In step 110, the risk presentation unit 30 presents the chronological prediction line 53 in addition to the chronological transition line 52 and the risk line 54. In step 111, the score calculation unit 23 calculates a score 55 indicating the future dementia risk of the user based on the result of a comparison between the pseudo first-age user brain health data 18a on the chronological prediction line 53 and the sample brain health data 17a associated with the individual characteristics 17b corresponding to individual characteristics obtained by including the first age along with the user individual characteristic 43 and with the lifestyle characteristics 17c corresponding to the user lifestyle characteristic 45. The risk presentation unit 30 presents the score 55 or a symbol corresponding to the score together with or in place of the chronological prediction line 53. In step 108, if the user lifestyle characteristic 45 has not been inputted, in step 110, the score calculation unit 23 calculates the score 55 based on the sample brain health data 17a associated with the individual characteristics 17b.

In step 112, the presentation system 10 determines whether a different (next) user lifestyle characteristic 45 has been provided. If a different user lifestyle characteristic 45 has been provided, the processing returns to step 108 to acquire this different lifestyle characteristic (or "second user lifestyle characteristic") 45, and the same processing as described above is performed. In step 109, the second chronological prediction line 53a relating to the second user lifestyle characteristic 45 is predicted, and in step 110, the first chronological prediction line 53 relating to the previous lifestyle characteristic (or "first user lifestyle characteristic") 45 and the second chronological prediction line 53a relating to the second user lifestyle characteristic 45 are presented side by side or consecutively to allow comparison. In step 111, a second score 55 on the second chronological prediction line 53a relating to the second user lifestyle characteristic 45 is calculated, and is presented together with or in place of the chronological prediction lines 53 and 53a in a state that enables comparison with the first score 55 on the first chronological prediction line 53 relating to the first user lifestyle characteristic 45.

In step 112, when a next user lifestyle characteristic has not been inputted, in step 113, it is determined whether a plurality of predictions are being made and when multiple predictions are not being made, in step 114, the risk presentation unit 30 generates the report 50 that includes the chronological transition line 52, the chronological prediction line 53, the risk line 54, the score 55, and the like and provides the report 50 to the user. If a plurality of chronological prediction lines 53 have been generated, in step 115, the risk presentation unit 30 creates a report 50 including the plurality of simulated chronological prediction lines 53, a plurality of scores 55, and the like and provides the report 50 to the user.

Note that in this embodiment, when scoring the dementia risk, brain state data and cognitive ability data are used having referred to the user lifestyle characteristics. This is performed to recognize a score (and in turn, a dementia risk) that is highly reliable. However, the scoring method according to the present invention is not limited to this method, and it is sufficient to perform scoring based on the result of a comparison between the user brain state data and the sample brain state data associated with individual characteristics corresponding to the user individual characteristic. As examples, it is not necessary to refer to lifestyle characteristics, and it is possible to use only brain state data without using cognitive ability data.

In addition, in cases like the report 50 depicted in Fig. 2 where a chronological information display area 51 is provided together with the score display area 57 and the dementia risk is displayed based on the brain state data in the chronological information display area 51, the score 55 or the like to be displayed in the score display area 57 may be found by referring only to the cognitive ability data.

As described above, the presentation system 10 recognizes the dementia risk by comparing brain health data including data relating to the state of the brain of the user (user bran state data) whose risk is to be determined and data relating to the states of the brains of sample subjects (sample brain state data, sampled brain state data). The data relating to the state of the brain is direct data in the form of quantitative parameters based on states of the brain that affect cognitive ability. That is, this dementia risk presentation system 10 is capable of using direct data in the form of data relating to the state of the brain and not indirect data, such as brain function that has been inferred from the user's brain waves and movements as in a conventional system.

By doing so, in this presentation system 10, when recognizing the dementia risk, the estimation accuracy for brain function has little effect. As a result, according to this presentation system 10, the dementia risk can be recognized more accurately compared to a conventional system.

In addition, in this presentation system 10, the recognized dementia risk can be presented to the user and others in the form of the result of a comparison between the user brain state data and the sample brain state data. By doing so, according to the presentation system 10, the user and others can intuitively grasp the scale of the dementia risk even when they do not have sufficient knowledge. The presentation system 10 can also present the dementia risk in a format that where a chronological transition line, which is a line in keeping with age, is compared with a risk line. This means that the user and others can grasp not only the scale of the dementia risk of the user but simultaneously also the trend in the dementia risk of the user (such as whether the risk is worsening, improving, or deterioration is being suppressed). In addition, it is possible to present the chronological prediction line, which is a prediction of transitions in dementia risk from the present to the future, so as to allow comparison with a risk line, which makes it possible for the user and others to more accurately grasp the trend in the dementia risk of the user.

Further, when recognizing the chronological prediction line, the presentation system 10 can refer to one or more user lifestyle characteristics and recognize a highly reliable chronological prediction line (and in turn the trend of the dementia risk), which makes it possible for the user and others to more accurately grasp the trend in the dementia risk of the user. Furthermore, by presenting chronological prediction lines relating to a plurality of user lifestyle characteristics in a form that allows comparison, it becomes possible for the user and others to intuitively grasp variations in the chronological prediction lines (and in turn in the dementia risk) in keeping with variations in lifestyle characteristics. By doing so, since the user and others can grasp the lifestyle characteristics that should be improved in specific terms, this provides an incentive to improve the lifestyle characteristics.

The presentation system 10 may present the dementia risk as a score or the like. When recognizing a score or the like, the user and others can recognize a highly reliable score or the like (and in turn dementia risk) by referring to the lifestyle characteristics of the user. By doing so, the user and others can more accurately grasp the trend in the dementia risk of the user. In addition, since the user and others can grasp lifestyle characteristics that should be improved in specific terms, this provides an incentive to improve the lifestyle characteristics.

The description given above discloses a dementia risk presentation system that presents the dementia risk of the user, including: a data storage unit in which multiple sets of sample brain state data, which are the data relating to the states of the brains of sample subjects, are stored in association with individual characteristics including at least one of the age, gender, and physical information of the sample subjects; a user brain state recognition unit that recognizes user brain state data, which is data relating to the state of the brain of the user; a user individual characteristics recognition unit that recognizes the user individual characteristics, which are individual characteristics of the user; a risk recognition unit that compares the user brain state data with the sample brain state data associated with individual characteristics corresponding to the user individual characteristic; and a risk presentation unit that presents the results of the comparison by the risk recognition unit. The sample brain state data and the user brain state data may be data indicating the volume of the entire brain or the volume of at least one predetermined region of the brain, the risk recognition unit may recognize, based on the past user brain state data and the present user brain state data, a chronological transition line indicating transitions in the user brain state data for the user from the past to the present, and may recognize a risk line determined by referring to age-related transitions in the sample brain state data based on the sets of the sample brain state data associated with the individual characteristics corresponding to the user individual characteristics, and the risk presentation unit may present the chronological transition line (aging line) and the risk line side by side to allow comparison.

The risk recognition unit may, in addition to recognizing the chronological transition line and the risk line, recognize an chronological prediction line that indicates transitions of the user brain state data predicted for the user from the present to the future based on the chronological transition line, and the risk presentation unit may present the chronological transition line, the chronological prediction line, and the risk line side by side so as to allow comparison. The system may include a user lifestyle characteristics recognition unit that recognizes a user lifestyle characteristic which is a lifestyle characteristic including at least one of the lifestyle habits and living environment of the user, the data storage unit may store the multiple sets of the sample brain state data in association with the individual characteristics and also the lifestyle characteristics of the sample subjects, and the risk recognition unit may recognize a trend line indicating age-related transitions in the multiple sets of the sample brain state data based on the sample brain state data associated with lifestyle characteristics corresponding to the user lifestyle characteristic and may recognize the chronological prediction line based on the trend line and the chronological transition line. When a user lifestyle characteristic changes, the risk recognition unit may recognize the chronological prediction line once again based on the changed user lifestyle characteristic, and the risk presentation unit may place the chronological prediction line recognized based on the user lifestyle characteristic before the change and the chronological prediction line recognized based on the user lifestyle characteristic after the change side by side to allow comparison or may alternatively present the lines consecutively to allow comparison.

The risk recognition unit may recognize the dementia risk of the user as a score, based on the result of a comparison between the user brain state data and the sample brain state data that are associated with individual characteristics corresponding to the user individual characteristics, and the risk presentation unit may present the score or a symbol corresponding to the score. The system may include a user lifestyle characteristics recognition unit that recognizes user lifestyle characteristics, which are lifestyle characteristics of the user, the data storage unit may store the sample brain state data in association with the individual characteristics and lifestyle characteristics including at least one of the lifestyle habits and living environment of the sample subjects, and the risk recognition unit may recognize the dementia risk of the user as a score based on the result of a comparison between the user brain state data, sample brain state data associated with individual characteristics corresponding to the user individual characteristics, and sample brain state data associated with lifestyle characteristics corresponding to the user lifestyle characteristics.

The system may be equipped with a user lifestyle characteristics recognition unit that recognizes user lifestyle characteristics, which are lifestyle characteristics of the user, the data storage unit may store the sample brain state data in association with the individual characteristics and lifestyle characteristics including at least one of the lifestyle habits and living environment of the sample subjects, the risk recognition unit may recognize, when a user lifestyle characteristic has changed, the score once again based on the changed user lifestyle characteristic, and the risk presentation unit may present the score or a symbol corresponding to the score that has been recognized based on the user lifestyle characteristic before the change and the score or a symbol corresponding to the score that has been recognized based on the user lifestyle characteristic after the change side by side to allow comparison or as an alternative consecutively to allow comparison.

The above description also discloses a dementia risk presentation method that presents the dementia risk of a user including: a step in which a data storage unit stores sample brain state data, which are data relating to the states of the brain of sample subjects, in association with individual characteristics including at least one of the age, gender, and physical information of the sample subjects; a step in which a user brain state recognition unit recognizes user brain state data, which is data relating to the state of the brain of the user; a step in which a user individual characteristic recognition unit recognizes user individual characteristics, which are individual characteristics of the user; a step in which a risk recognition unit compares the user brain state data with the sample brain state data associated with individual characteristics corresponding to the user individual characteristics; and a step in which a risk presentation unit presents the result of the comparison by the risk recognition unit.

Although the present invention has been described by way of the illustrated embodiments, the present invention is not limited to these embodiments. As one example, in the presentation system of the above embodiment, a value indicating hippocampal volume is used as data relating to the brain state. In addition, when determining the dementia risk, the user lifestyle characteristics are referred to in addition to the user individual characteristics. However, the dementia risk presentation system according to the present invention is not limited to this configuration and may be any configuration that determines the dementia risk by comparing brain state data based on individual characteristics. As examples, instead of hippocampal volume, values relating to the entire brain or other regions may be used, and no reference may be made to lifestyle characteristics. In addition, other changes that could be conceived by those skilled in the art are included in the scope of the present invention as defined by the range of the patent claims.

**Claims**

1. A system comprising:

    a first database that includes sample brain health data of sample subjects associated with individual characteristics including at least one of age, gender, and physical information of the sample subjects respectively, the sample brain health data including at least one type of data for sample brain state data that are data pertaining to states of brains of the sample subjects and sample cognition data that are data pertaining to cognitive abilities as functions of the brains of the sample subjects;
    a first recognition unit that recognizes user brain health data including at least one type of data for user brain state data, which is data pertaining to a state of a brain of a user, and user cognition data, which is data pertaining to cognitive ability as a function of the brain of the user;
    a second recognition unit that recognizes a user individual characteristic indicating individual characteristics of the user; and
    a risk presentation unit that presents a chronological transition line and a risk line relating to dementia, the chronological transition line indicating transitions in the user brain health data of the user from past to present based on past and present user brain health data of the user, the risk line being derived by referring to transitions according to age in the sample brain health data associated with the individual characteristics corresponding to the user individual characteristic.

**2.** The system according to claim 1,
wherein the risk presentation unit includes a first score presentation unit that presents a score indicating dementia risk of the user or a symbol corresponding to the score based on a result of a comparison between the user brain health data and the sample brain health data associated with individual characteristics corresponding to the user individual characteristic.

**3.** The system according to claim 1 or 2,
further comprising a prediction unit that predicts a chronological prediction line indicating predicted transitions of the user brain health data of the user from present to future based on the chronological transition line,
wherein the risk presentation unit includes a prediction presentation unit that presents the chronological prediction line in addition to the chronological transition line and the risk line.

**4.** The system according to claim 3,
wherein the risk presentation unit includes a second score presentation unit that presents, together with the chronological prediction line or in place of the chronological prediction line, a score indicating a future dementia risk of the user or a symbol corresponding to the score, based on a result of a comparison between the user brain health data at a first age on the chronological prediction line and the sample brain health data associated with individual characteristics corresponding to an individual characteristic obtained by including the first age along with the user individual characteristic.

**5.** The system according to claim 3 or 4,
wherein in the first database, the sample brain health data of the sample subjects are associated with the individual characteristics and with lifestyle characteristics of the sample subject, the lifestyle characteristics including at least one of lifestyle habits and living environments of the sample subjects,
the system further includes a third recognition unit that recognizes a user lifestyle characteristic indicating lifestyle characteristics of the user, and
the prediction unit predicts the chronological prediction line based on the chronological transition line and the sample brain health data associated with the individual characteristics corresponding to the user individual characteristic, the lifestyle characteristics corresponding to the user lifestyle characteristic, and age of the user.

**6.** The system according to claim 5,
wherein the risk presentation unit includes a third score presentation unit that presents, together with or instead of the chronological prediction line, a score indicating the future dementia risk of the user or a symbol corresponding to the score, based on a result of a comparison between the user brain health data at a first age on the chronological prediction line and the sample brain health data associated with individual characteristics corresponding to an individual characteristic obtained by including the first age along with the user individual characteristic and the lifestyle characteristics corresponding to the user lifestyle characteristic.

**7.** The system according to claim 5 or 6,
wherein the prediction unit includes a comparison/prediction unit that recognizes a first chronological prediction line based on a first user lifestyle characteristic and a second chronological prediction line based on a second user lifestyle characteristic for an identical user, and
the risk presentation unit includes a comparison/presentation unit that presents the first chronological prediction line and the second chronological prediction line side by side or consecutively to allow comparison.

**8.** The system according to claim 7,
wherein the risk presentation unit including a fourth score presentation unit that presents, together with or in place of the first chronological prediction line and the second chronological prediction line:

a first score indicating a future dementia risk of the user or a symbol corresponding to the first score based on a result of a comparison between the user brain health data of a first age on the first chronological prediction line and the sample brain health data associated with individual characteristics corresponding to an individual characteristic obtained by including the first age along with the user individual characteristic and a lifestyle characteristic corresponding to the first user lifestyle characteristic; and
a second score indicating the future dementia risk of the user or a symbol corresponding to the second score based on a result of a comparison between the user brain health data of the first age on the second chronological prediction line and the sample brain health data associated with individual characteristics corresponding to an individual characteristic obtained by including the first age along with the user individual characteristics and a lifestyle characteristic corresponding to the second user lifestyle characteristic.

9.  The system according to any one of claims 1 to 8,
    wherein the sample brain state data and the user brain state data include at least one type of data volumes of entire brains, volumes of at least one predetermined regions of the brains, brain images, electroencephalograms, and cerebral blood flows.

10. A method of presenting dementia risk using a system capable of referring to a first database in which sample brain health data of sample subjects, including at least one type of data for sample brain state data that are data pertaining to states of brains of the sample subjects and sample cognition data that are data pertaining to cognitive ability as functions of the brains of the sample subject, are associated with individual characteristics including at least one of age, gender, and physical information of the sample subjects respectively, the method comprising:

    recognizing user brain health data including at least one type of data for user brain state data, which is data pertaining to a state of a brain of a user, and user cognition data, which is data pertaining to cognitive ability as a function of the brain of the user;
    recognizing a user individual characteristic indicating individual characteristics of the user; and
    presenting a chronological transition line and a risk line relating to dementia, the chronological transition line indicating transitions in the user brain health data of the user from past to present based on past and present user brain health data of the user, the risk line being derived by referring to transitions according to age in the sample brain health data associated with the individual characteristics corresponding to the user individual characteristic.

11. The method according to claim 10,
    further comprising presenting a score indicating dementia risk of the user or a symbol corresponding to the score based on a result of a comparison between the user brain health data and the sample brain health data associated with individual characteristics corresponding to the user individual characteristic.

12. The method according to claim 10 or 11,
    further comprising predicting a chronological prediction line indicating predicted transitions of the user brain health data for the user predicted from the present to future based on the chronological transition line,
    wherein presenting the risk includes presenting the chronological prediction line in addition to the chronological transition line and the risk line.

13. The method according to claim 12,
    further comprising presenting, together with the chronological prediction line or in place of the chronological prediction line, a score indicating a future dementia risk of the user or a symbol corresponding to the score, based on a result of a comparison between the user brain health data at a first age on the chronological prediction line and the sample brain health data associated with individual characteristics corresponding to an individual characteristic obtained by including the first age along with the user individual characteristic.

14. The method according to claim 12 or 13,
    wherein in the first database, the sample brain health data of the sample subject are associated with the individual characteristics and with lifestyle characteristics of the sample subjects, the lifestyle characteristics including at least one of lifestyle habits and living environments of the sample subjects,
    the method further comprises recognizing a user lifestyle characteristic indicating lifestyle characteristics of the user, and
    the predicting includes predicting the chronological prediction line based on the chronological transition line and the sample brain health data associated with the individual characteristics corresponding to the user individual characteristic, the lifestyle characteristics corresponding to the user lifestyle characteristic, and age of the user.

15. The method according to claim 14,
    wherein the method further comprises presenting, together with or instead of the chronological prediction line, a score indicating the future dementia risk of the user or a symbol corresponding to the score, based on a result of a comparison between the user brain health data at a first age on the chronological prediction line and the sample brain health data associated with individual characteristics corresponding to an individual characteristic obtained by including the first age along with the user individual characteristic and the lifestyle characteristics corresponding to the user lifestyle characteristic.

**16.** The method according to claim 14 or 15,
wherein the predicting includes predicting a first chronological prediction line based on a first user lifestyle characteristic and a second chronological prediction line based on the second user lifestyle characteristic for the identical user, and
the presenting of the risk includes presenting the first chronological prediction line and the second chronological prediction line side by side or consecutively to allow comparison.

**17.** The method according to claim 16,
further comprising presenting, together with or in place of the first chronological prediction line and the second chronological prediction line:

a first score indicating a future dementia risk of the user or a symbol corresponding to the first score based on a result of a comparison between the user brain health data of a first age at the first chronological prediction line and the sample brain health data associated with individual characteristics corresponding to an individual characteristic obtained by including the first age along with the user individual characteristic and a lifestyle characteristic corresponding to the first user lifestyle characteristic; and
a second score indicating the future dementia risk of the user or a symbol corresponding to the second score based on a result of a comparison between the user brain health data of the first age at the second chronological prediction line and the sample brain health data associated with individual characteristics corresponding to an individual characteristic obtained by including the first age along with the user individual characteristic and a lifestyle characteristic corresponding to the second user lifestyle characteristic.

**18.** A program comprising instructions that cause a computer to operate as the system according to any one of claims 1 to 9.

# Fig. 1

# Fig. 2

50

51

Hippocampal volume mm³

8,000
7,500
7,000
6,500
6,000
5,500
5,000

Normal range 54a

54

Caution zone 54b

Age

53

52

41yrs  45yrs  50yrs

57

Overall Risk Determination

B

Your brain age is **50** years old (equivalent)  56a

Long-term Prediction
There is a risk of drop  56b

A --- B --- C --- D --- F  56c

58

Lifestyle Characteristics

How often do you exercise?
☐Never  ☑Once a week  ☐2 or 3 times a week  ☐Daily

How often do you drink alcohol?
☐Never  ☐Once a week  ☑2 or 3 times a week  ☐Daily

How much do you smoke?
☑Never  ☐Up to 10 a day  ☐Over 10 a day

55

59

# Fig. 3

Hippocampal volume
mm³

Transitions in hippocampal volume for age (male)

Age

# Fig. 4

Hippocampal volume
mm³

67a   67   67a

Transitions in hippocampal volume for age (male)

53w   Age

## Fig. 5

## Fig. 6

# Fig. 7

**(a)**

50

55 → Overall Risk Determination

**B**

56b → Your brain age is **50** years old(equivalent) ← 56a

A---B---C---D---F ← 56c

Long-term Prediction
There is a risk of drop

58 → Lifestyle Characteristics

How often do you exercise?
☐Never ☑Once a week ☐2 or 3 times a week ☐Daily

How often do you drink alcohol?
☐Never ☐Once a week ☐2 or 3 times a week ☑Daily

How much do you smoke?
☑Never ☐Up to 10 a day ☐Over 10 a day

**(b)**

50

55 → Overall Risk Determination

**A**

56b → Your brain age is **47** years old(equivalent) ← 56a

A---B---C---D---F ← 56c

Long-term Prediction
The is little risk of drop

58 → Lifestyle Characteristics

How often do you exercise?
☐Never ☑Once a week ☐2 or 3 times a week ☐Daily

How often do you drink alcohol?
☐Never ☐Once a week ☑2 or 3 times a week ☐Daily

How much do you smoke?
☑Never ☐Up to 10 a day ☐Over 10 a day

# Fig. 8

50

Detailed Brain Function

57

**Present Brain Function**

# A

55

78

Short-term
memory

● Present results
⬤ Standard in the
same age group

76

**CQ score**

# 116

Score Range (55 to 145)

Calculation

Working memory

Comparison with
the same age group

Standard in the same age group

B

C

A 77

D

55    100    145

116

Spatial cognition    Executive function

74a

75

72a

56a

Transitions
in brain functions

74b

Short-term memory

72b

Working memory

74c

72c

Brain age

50s

74d

Executive function

72d

Standard in the
same age group

74e

Spatial cognition

72e

71

Calculation

2017
12/20

2019
1/15

2019
6/13

51

Changes in hippocampal volume

Your hippocampal volume is shrinking faster than
standard person

● Your hippocampal volume ⬤ Average for the same age group

Your hippocampus shrinking trend is
faster than that of standard person.
Improve your lifestyle characteristics
to prevent a loss in brain function.

52

54

65

2019
1/15

2019
6/13

□【white】 Standard ■【red】 Caution

# Fig. 9

| Test/Investigation type | Name |
|---|---|
| Cognitive ability test | MEDE (Multifaceted Early Dementia Judgment Examination) |
| Cognitive ability test | WHO QOL26 |
| Cognitive ability test | COGNISTAT |
| Cognitive ability test | SIB |
| Cognitive ability test | Coghealth |
| Cognitive ability test | NPI |
| Cognitive ability test | BEHAVE-AD |
| Cognitive ability test | Reading aloud test |
| Cognitive ability test | HDS-R (Hasegawa's Dementia Scale-Revised) |
| Cognitive ability test | MMSE (Mini-Mental State Examination) |
| Cognitive ability test | Frontal Assessment Battery |
| Cognitive ability test | Stroop Test |
| Cognitive ability test | MoCA (Montreal Cognitive Assessment) |
| Cognitive ability test | Mini-cog |
| Cognitive ability test | WMS-R (Wechsler Memory Test) |
| Cognitive ability test | DASC-21 (Dementia Assessment Sheet for Community-based Integrated Care System-21 items) |
| Cognitive ability test | RBMT (Rivermead Behavioral Memory Test) |
| Cognitive ability test | ADAS-Cog |
| Cognitive ability test | Logical Memory |
| Cognitive ability test | Rey Auditory Verbal Learning Test |
| Cognitive ability test | Clock Drawing |
| Cognitive ability test | Category Fluency |
| Cognitive ability test | Trails Making Test |
| Cognitive ability test | Boston Naming Test |
| Cognitive ability test | Clinical Dementia Rating (CDR) |
| Cognitive ability test | IADL |
| Cognitive ability test | JAGES |
| Cognitive ability test | Kohs block design test |
| Cognitive ability test | CANTAB |

# Fig. 10

| Brain or mental illness test | CAS (Clinical Anxiety Scale test) |
|---|---|
| Brain or mental illness test | SDS (Self-rating Depression Scale self-assessment) |
| Brain or mental illness test | CES-D (Center for Epidemiologic Studies Depression Scale self-assessment) |
| Brain or mental illness test | HDRS (Hamilton Depression Rating Scale) |
| Brain or mental illness test | STAI (State-Trait Anxiety Inventory test) |
| Brain or mental illness test | POMS |
| Brain or mental illness test | IES-R |
| Brain or mental illness test | PDS |
| Brain or mental illness test | TK diagnostic new parent-child relationship test |
| Brain or mental illness test | CMI health questionnaire |
| Brain or mental illness test | GHQ Mental health evaluation sheet |
| Brain or mental illness test | MAS Manifest Anxiety Scale |
| Brain or mental illness test | Bourdon' seher Durchstreichte |
| Brain or mental illness test | LSAS-J (Japanese version) |
| Brain or mental illness test | DES- Ⅱ |
| Brain or mental illness test | EAT-26 |
| Brain or mental illness test | DSRS-C |
| Brain or mental illness test | K6 |
| Brain or mental illness test | WH05 |
| Brain or mental illness test | Occupational stress simple survey |
| Brain or mental illness test | GDS |

# Fig. 11

START

ACQUIRE USER BRAIN HEALTH DATA
(USER BRAIN STATE/USER COGNITIVE ABILITY)
FROM THE PAST TO THE PRESENT — 101

ACQUIRE A USER INDIVIDUAL CHARACTERISTIC — 102

103
IS PREDICTION REQUIRED? — N — → 104

PRESENT CHRONOLOGICAL
TRANSITION LINE AND
DEMENTIA RISK LINE OF THE USER

Y

107
HAS A USER
LIFESTYLE CHARACTERISTIC
BEEN INPUTTED? — N

105

PRESENT THE SCORE OF
DEMENTIA RISK OF THE USER

Y

ACQUIRE THE USER LIFESTYLE
CHARACTERISTIC — 108

106

CALCULATE CHRONOLOGICAL
PREDICTION LINE — 109

OUTPUT A REPORT THAT INCLUDES
CHRONOLOGICAL TRANSITION LINE,
RISK LINE, AND THE SCORE

PRESENT CHRONOLOGICAL TRANSITION LINE,
CHRONOLOGICAL PREDITION LINE,
AND RISK LINE — 110

PRESENT THE SCORE — 111

112
HAS A DIFFERENT
Y — USER LIFESTYLE CHARACTERISTIC
BEEN INPUTTED?

N

113
IS THERE A PLURALITY
OF PREDICTIONS? — N → 114

OUTPUT A REPORT THAT INCLUDES
CHRONOLOGICAL TRANSITION LINE,
CHRONOLOGICAL PREDITION LINE,
RISK LINE, AND THE SCORE

Y

OUTPUT A REPORT THAT
INCLUDES A PLURALITY OF
CHRONOLOGICAL PREDITIONS

115

END

<div align="center">

**INTERNATIONAL SEARCH REPORT**

</div>

| | International application No. |
|---|---|
| | PCT/JP2019/030056 |

**A. CLASSIFICATION OF SUBJECT MATTER**

Int.Cl. A61B5/055(2006.01)i, A61B6/03(2006.01)i, A61B10/00(2006.01)i, G06Q50/22(2018.01)i

According to International Patent Classification (IPC) or to both national classification and IPC

**B. FIELDS SEARCHED**

Minimum documentation searched (classification system followed by classification symbols)
Int.Cl. A61B5/055, A61B6/03, A61B10/00, G06Q50/22

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
メディカルオンライン

**C. DOCUMENTS CONSIDERED TO BE RELEVANT**

| Category* | Citation of document, with indication, where appropriate, of the relevant passages | Relevant to claim No. |
|---|---|---|
| X | JP 2017-124039 A (TOSHIBA MEDICAL SYSTEMS CORPORATION) 20 July 2017, paragraphs [0019]-[0027], [0045]-[0056], fig. 7-9 & US 2017/0206654 A1, paragraphs [0028]-[0036], [0054]-[0065], fig. 7-9 | 1,2,9-10,12,18 |
| Y | | 2,4,11,13 |
| Y | 松田博史，MRI 標準データベースを使用したアルツハイマー型痴呆の早期診断を考える，老年精神医学雑誌，vol. 16, 増刊－3, December 2005, pp. 38-44, non-official translation (MATSUDA, Hiroshi, "Considering the early diagnosis of Alzheimer-type dementia using MRI standard database", Japanese journal of geriatric psychiatry, special issue-3) | 2,4,11,13 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 October 2019 (21.10.2019) | 29 October 2019 (29.10.2019) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2019/030056

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2015-84970 A (ARAYA BRAIN IMAGING INC.) 07 May 2015, entire text, all drawings & US 2016/0155226 A1, whole document & WO 2015/064665 A1 & EP 3064126 A1 | 1-18 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016022310 A

- JP 2018033652 A